# DEMANDE DE BREVET EUROPEEN

(11) **EP 1 695 693 A1**
(43) Date de publication de la demande: **30.08.2006**
(21) Numéro de dépôt: 06300087.1
(22) Date de dépôt: 31.01.2006
(51) Int. Cl.: A61K 8/898

(54) **Composition cosmétique solide de maquillage et/ou de soin**

(30) Priorité: 31.01.2005 FR 0550266
(71) Demandeur: L'ORÉAL, 75008 Paris (FR)
(72) Inventeur: Blin, Xavier, 75015 Paris (FR); Ferrari, Véronique, 94700 Maisons-Alfort (FR); Paute, Chrystel, 94100 Saclay (FR)
(74) Mandataire: Le Coupanec, Pascale A.M.P.

(57) **Abrégé**

La présente invention concerne une composition cosmétique solide caractérisée en ce qu'elle comprend au moins une matière colorante et une phase grasse liquide texturée par une quantité efficace d'au moins un copolymère organique et en ce qu'elle présente une dureté variant de 10 à 250 g et une élasticité supérieure à 80 %.

## Description

La présente invention se rapporte à une composition cosmétique solide notamment de maquillage et/ou de soin destinée à être appliquée sur la peau, les lèvres comprenant au moins une phase grasse liquide texturée par une quantité efficace d'au moins un copolymère organique.

Dans les produits cosmétiques ou dermatologiques, il est courant de trouver une phase grasse liquide structurée, à savoir gélifiée et/ou rigidifiée. Ceci est notamment le cas dans les compositions solides, en particulier les compositions coulées solides, les baumes et les rouges à lèvres, les fards à paupières, les produits anti-cernes et les fonds de teint coulés. Cette structuration est classiquement obtenue à l'aide de cires ou de charges ou plus récemment à partir de gélifiants spécifiques.

Dans les documents WO-A-97/36573, US-A-5 874 069, US-A-5 919 441, US-A-6 051 216, WO-A-02/17870, WO-A-02/17871, EP-A-1 177 784, WO-A-99/06473, et US-A-6 353 076 qui est une division du US-A-6 051 216, sont proposées des compositions cosmétiques telles que des sticks ou des gels de déodorant, comprenant une phase huileuse siliconée gélifiée par un polymère polysiloxane/polyamide.

La présente invention résulte plus particulièrement de l'observation par les inventeurs que l'utilisation de copolymère(s) organique(s) permet d'accéder à une texture solide originale de composition cosmétique, à savoir dotée d'une rigidité relativement faible et d'une élasticité élevée. Cette texture ne correspond ni à celle d'un stick de l'art antérieur doté d'une rigidité assez élevée, ni à celle d'un gel dont la consistance est liquide ou pâteuse.

En conséquence, la présente invention concerne selon un premier aspect, une composition cosmétique solide caractérisée en ce qu'elle comprend au moins une matière colorante et une phase grasse liquide texturée par une quantité efficace d'au moins un copolymère organique et en ce qu'elle présente une dureté variant de 10 à 250 g et une élasticité supérieure à 80 %.

Elle concerne en outre une composition cosmétique solide caractérisée en ce qu'elle comprend au moins une phase grasse liquide texturée par une quantité efficace d'au moins deux copolymères organiques distincts et en ce qu'elle présente une dureté variant de 10 à 250 g et une élasticité supérieure à 80 %

La présente invention a également pour objet une composition cosmétique solide caractérisée en ce qu'elle comprend une phase grasse liquide comprenant au moins une huile siliconée, ladite phase grasse étant texturée par une quantité efficace d'au moins un copolymère organique et en ce qu'elle présente une dureté variant de 10 à 250 g et une élasticité supérieure à 80 %.

Elle concerne de même selon un autre de ses aspects, une composition cosmétique solide caractérisée en ce qu'elle comprend une phase grasse liquide texturée par une quantité efficace d'au moins un copolymère organique et au moins un tensioactif siliconé et en ce qu'elle présente une dureté variant de 10 à 250 g et une élasticité supérieure à 80 %. Plus particulièrement, ce copolymère est choisi parmi :
1) des copolymères hybrides comportant au moins deux motifs capables d'établir des interactions hydrogène, ces deux motifs étant situés dans la chaîne du copolymère, notamment un polyorganosiloxane comportant au moins deux motifs capables d'établir des interactions hydrogènes, ces deux motifs étant situés dans la chaîne du copolymère, et/ou
2) des copolymères hybrides comportant au moins deux motifs capables d'établir des interactions hydrogènes, ces deux motifs étant situés sur des greffons ou ramifications, notamment un polyorganosiloxane comportant au moins deux motifs capables d'établir des interactions hydrogènes, ces deux motifs étant situés sur des greffons
ou ramifications.

La présente invention a également pour objet une composition cosmétique solide caractérisée en ce qu'elle comprend une phase grasse liquide ladite phase grasse étant texturée par une quantité efficace d'au moins deux copolymères différents de formule générale I 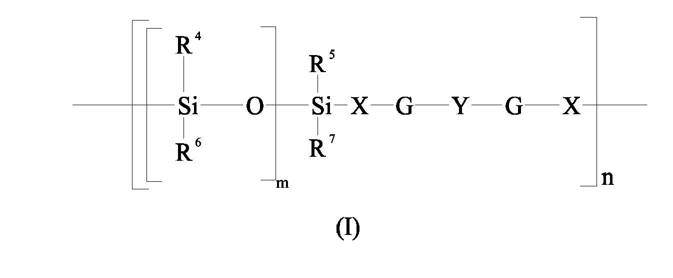 les deux copolymères possédant des indices m de valeurs différentes, l'un étant supérieur ou égal à 50 et l'autre inférieur ou égal à 50.

Plus particulièrement, une composition telle que définie ci-dessus peut comprendre au moins :
- 1 à 90 % en poids d'au moins une huile siliconée,
- 5 à 50 % en poids d'au moins un copolymère possédant un indice m de valeur supérieure ou égale à 50, et notamment égale à 100,
- 0,1 à 10 % en poids d'au moins un copolymère possédant un indice m de valeur inférieure ou égale à 50, et notamment égale à 15,
- 0,1 à 10 % en poids d'un tensioactif siliconé, et
- 0,001 à 40 % en poids d'au moins une matière colorante.

La présente invention a également pour objet une composition cosmétique destinée au maquillage des lèvres et notamment un rouge à lèvres caractérisée en ce qu'elle comprend une phase grasse liquide texturée par au moins un copolymère de formule générale I telle que définie ci-dessus dans laquelle m est supérieur ou égal à 50.

L'invention a également pour objet l'utilisation d'un copolymère de formule générale (I) telle que définie ci-dessus, dans laquelle m est supérieur ou égal à 50, dans une composition cosmétique de maquillage des lèvres, par exemple un produit de rouge à lèvres.

Elle concerne également selon un autre de ses aspects, une composition cosmétique solide caractérisée en ce qu'elle comprend au moins une phase grasse liquide texturée par une quantité efficace d'au moins un copolymère éthylénique et en ce qu'elle présente une dureté variant de 10 à 250 g et une élasticité supérieure à 80 %.

Selon encore un autre de ses aspects, la présente invention a également pour objet un procédé de maquillage et/ou de soin de la peau et/ou des lèvres, comprenant l'application sur celles-ci d'au moins une composition cosmétique conforme à la présente invention.

Selon un autre de ses aspects, l'invention a pour objet un support synthétique sur lequel est présent, en tout ou partie de sa surface, au moins une couche d'une composition selon l'invention.

Selon un autre aspect, l'invention concerne également un ensemble cosmétique comprenant :
i) un récipient délimitant au moins un compartiment, ledit récipient étant fermé par un élément de fermeture ; et
ii) une composition disposée à l'intérieur dudit compartiment, la composition étant telle que définie précédemment.

Selon encore un autre de ses aspects, l'invention a pour objet l'utilisation d'au moins un copolymère organique, en association avec au moins une phase grasse liquide pour la préparation d'une composition cosmétique présentant une dureté variant de 10 à 250 g et une élasticité supérieure à 80 %.

Avantageusement, les compositions selon l'invention sont dotées de nouvelles textures qui peuvent être associées à de nouvelles gestuelles d'application, c'est notamment le cas pour des produits tels que les rouges à lèvres où le produit peut être directement appliqué sur les lèvres sans l'aide d'un applicateur.

Plus précisément, les compositions selon l'invention se présentent, non pas sous une forme solide dure à l'image d'un stick ou sous une forme fluide comme un gloss ou encore sous une forme de type pâte souple, mais sous la forme d'une texture mousse ou gomme dotée simultanément de propriétés souple et élastique.

Les textures solides souples et élastiques obtenues selon l'invention sont compatibles avec une application directe sur les lèvres sans l'utilisation d'applicateur comme dans le cas de textures fluides. Elles confèrent à l'application de la souplesse, de la douceur avec une très bonne élasticité préservant ainsi le produit pour une prochaine application. Le produit ne se déforme pas de façon définitive, mais reprend sa forme initiale après application.

Avantageusement, la composition conforme à l'invention, sous la forme d'un stick, a le comportement d'un solide élastique déformable et souple, conférant à l'application une douceur élastique remarquable.

Par phase grasse liquide, au sens de la demande, on entend une phase grasse liquide à température ambiante (25 °C) et pression atmosphérique (760 mm de Hg), composée d'un ou plusieurs corps gras liquides à température ambiante, appelés aussi huiles, compatibles entre eux.

Par "phase grasse liquide texturée", on entend une phase grasse dont la viscosité est augmentée par ajout du copolymère organique.

Par "composition solide", on désigne une composition qui ne s'écoule pas sous son propre poids. Cette capacité à ne pas s'écouler sous son propre poids peut en particulier être déterminée en plaçant la composition dans un récipient en verre transparent après avoir préalablement délimiter la zone sur laquelle est déposée l'échantillon. L'échantillon est ainsi laissé pendant environ 12 heures à une température de 25 °C. Au terme de ce délai, aucun écoulement en dehors de la zone de dépôt n'est observé à l'oeil nu.

Les compositions selon l'invention possèdent avantageusement une dureté variant de 10 à 250 g et une élasticité supérieure à 80 %.

### MESURE DE L'ELASTICITE ET DE LA DURETE

La dureté et l'élasticité de la composition selon l'invention peuvent être mesurées à l'aide d'un texturomètre qui permet d'obtenir la variation de la résistance à la déformation de la composition en fonction du déplacement d'un mobile dans un échantillon de ladite composition.

Le texturomètre mesure la force de résistance à la déformation de la composition dès que le mobile entre en contact avec l'échantillon. Après avoir atteint une profondeur maximale programmée L0 dans l'échantillon, le mobile retourne au point initial.

La dureté (exprimée en gramme ou en Newton) est égale à la valeur de résistance de la composition lorsque le mobile est en bout de course, et l'élasticité (exprimée en pourcentage) est égale au rapport de i) la distance L à laquelle se produit la rupture de contact entre le mobile et l'échantillon au cours du retrait du mobile et ii) de la distance L0. La rupture de contact se traduit par l'annulation de la force de résistance de la composition sur le mobile. L'élasticité est proportionnelle à la distance sur laquelle le système va "accompagner" la remontée du mobile; plus elle est importante, plus le système est élastique.

Le texturomètre utilisé peut notamment être un texturomètre Stable Micro System TAX-T2i® équipé du logiciel d'exploitation type Texture Expert Exceed® muni d'un mobile plastique hémisphérique P/0.5 HS Rheo's.

Les paramètres appliqués sont avantageusement les suivants :
- vitesse avant contact : 0,1 mm.s⁻¹,
- vitesse de déplacement dans l'échantillon : 0,1 mm.s⁻¹,
- vitesse de retrait : 0,1 mm.s⁻¹,
- profondeur maximale L0 : 1 mm.

On prépare les échantillons de composition en coulant à chaud une quantité suffisante de la composition à tester, par exemple dans une boite de Pétri 100 x 15 mm préalablement tarée, pour obtenir un échantillon d'environ 1 cm d'épaisseur. L'intérêt du choix de ce conditionnement est sa largeur suffisante pour s'affranchir de tout effet de bord. On prépare ainsi deux boîtes de Pétri, qui sont laissées au repos un minimum de 24 h à 20 °C avant caractérisation.

Au minimum trois mesures sont réalisées sur chaque échantillon: une, au centre et d'autres, sur des points situés à équidistance du centre et du bord de celle-ci.

La dureté et l'élasticité sont égales à la moyenne des mesures effectuées, au nombre minimum de six.

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Les compositions selon l'invention possèdent avantageusement une élasticité supérieure à 90 % et en particulier proche de 100 %.

Plus particulièrement, la rigidité peut varier de 30 g à 200 g, notamment de 50 g à 175 g et plus particulièrement de 75 g à 110 g. Comme précisé précédemment, ces propriétés physiques des compositions selon l'invention sont obtenues grâce à la texturation de leur phase grasse liquide par une quantité efficace d'au moins un copolymère organique dit encore "copolymère texturant" selon l'invention.

Les copolymères texturants peuvent être choisis en terme de nature et de quantité, selon la texture ou dureté souhaitée et la stabilité souhaitée des compositions et en fonction de l'application spécifique envisagée.

La quantité du (au moins un) copolymère texturant peut être telle qu'elle conduit à l'obtention d'un solide désintégrable qui ne s'écoule pas sous l'effet de son propre poids.

### Copolymère organique

Par "copolymère", on entend au sens de l'invention un composé obtenu par réaction de deux composés chimiques de structures différentes, dont un au moins est un composé organique. Ce copolymère peut être un copolymère hybride obtenu par réaction d'un monomère hydrocarboné et d'un monomère siliconé ou encore un copolymère éthylénique. Plus particulièrement, ce copolymère hybride peut être choisi parmi :
1) des copolymères hybrides comportant au moins deux motifs capables d'établir des interactions hydrogène, ces deux motifs étant situés dans la chaîne du copolymère, notamment un polyorganosiloxane comportant au moins deux motifs capables d'établir des interactions hydrogènes, ces deux motifs étant situés dans la chaîne du copolymère, et/ou
2) des copolymères hybrides comportant au moins deux motifs capables d'établir des interactions hydrogènes, ces deux motifs étant situés sur des greffons ou ramifications, notamment un polyorganosiloxane comportant au moins deux motifs capables d'établir des interactions hydrogènes, ces deux motifs étant situés sur des greffons
ou ramifications.

A titre représentatif des composés de la famille 1), on peut notamment citer les composés de formule I plus particulièrement décrits ci-après :

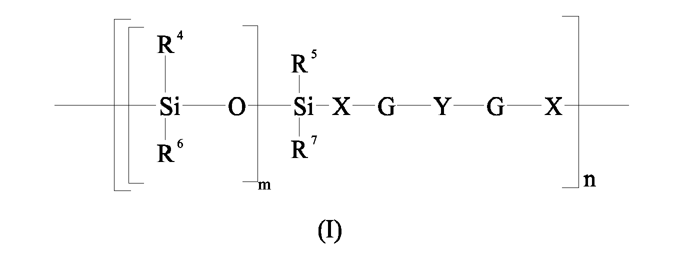

En ce qui concerne les composés de la famille 2), ils peuvent notamment être représentés par les composés de formule (II) plus particulièrement décrits ci-après.

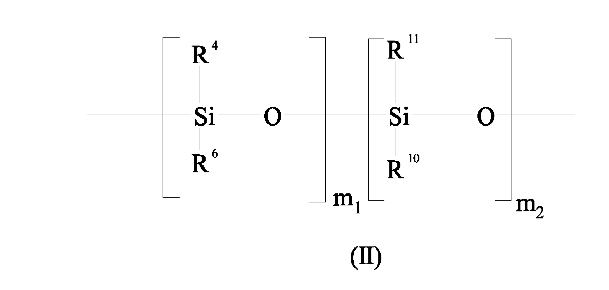

En ce qui concerne les motifs capables d'établir des liaisons hydrogénées, ils peuvent être choisis parmi les groupes ester, amide, sulfonamide, carbamate, thiocarbamate, urée, uréthane, thiourée, oxamido, guanidino, biguanidino et leurs combinaisons.

Dans la composition de l'invention, le copolymère organique, représente généralement de 0,5 à 80 %, notamment de 2 à 60 % en particulier de 5 à 40 % et plus particulièrement de 3 à 30% en poids par rapport au poids total de la composition.

Par ailleurs, le rapport massique copolymère texturant/phase grasse liquide de la composition est de préférence de 0,01 à 10, de préférence encore de 0,05 à 5, mieux de 0,1 à 2.

### COPOLYMERE HYBRIDE

Selon une première variante, les copolymères utilisés comme agents texturants dans la composition de l'invention peuvent être des polymères du type polyorganosiloxane tels que ceux décrits dans les documents US-A-5 874 069, US-A-5,919,441, US-A-6,051,216 et US-A-5,981,680.
a) Selon une première variante, il s'agit de polyorganosiloxane tels que définis ci-dessus et dont les motifs capables d'établir des interactions hydrogènes sont disposés dans la chaîne du copolymère.

Les polymères peuvent plus particulièrement être des polymères comprenant au moins un motif répondant à la formule générale I : 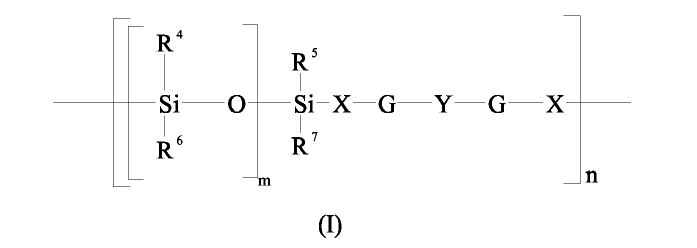 dans laquelle :
1) R⁴, R⁵, R⁶ et R⁷, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 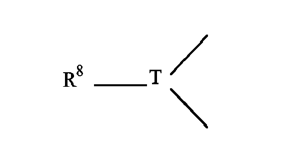 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S,
   ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 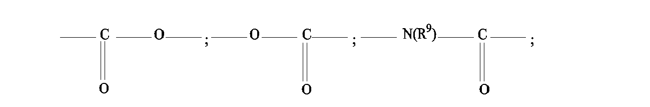 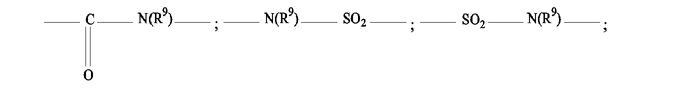 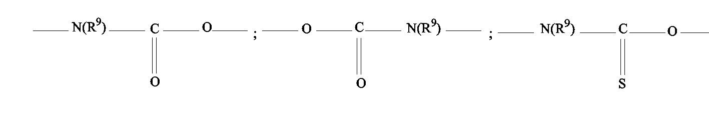 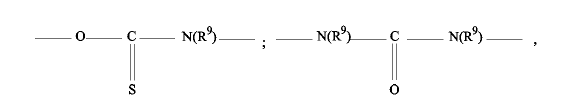 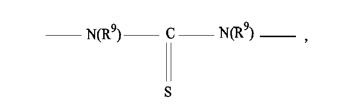 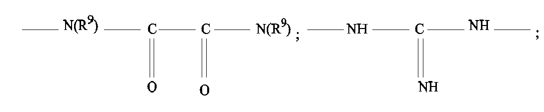 et 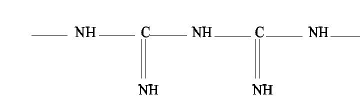 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 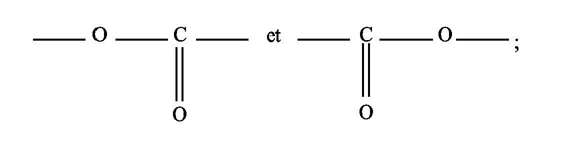
6) n est un nombre entier allant de 2 à 500, de préférence de 2 à 200, et m est un nombre entier allant de 1 à 1000, de préférence de 1 à 700 et mieux encore de 6 à 200.

Selon l'invention, 80 % des R⁴, R⁵, R⁶ et R⁷, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆,
g) les chaînes polyorganosiloxane de formule : 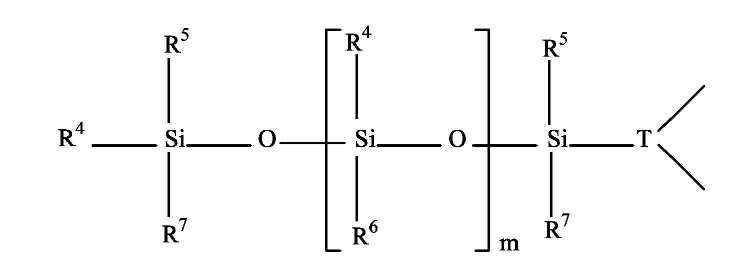 dans laquelle R⁴, R⁵, R⁶, R⁷, T et m sont tels que définis ci-dessus, et
h) les chaînes polyorganosiloxanes de formule : 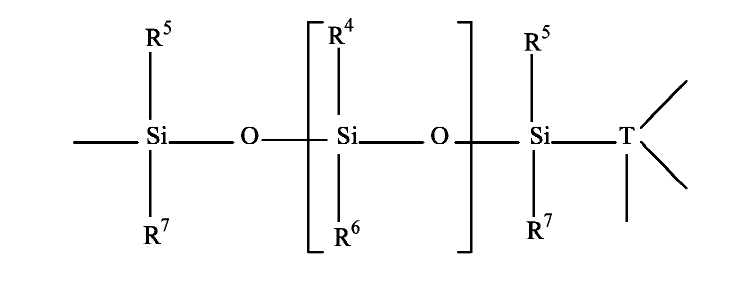
b) Selon la seconde variante, les polyorganosiloxanes peuvent être des polymères comprenant au moins un motif répondant à la formule (II) : 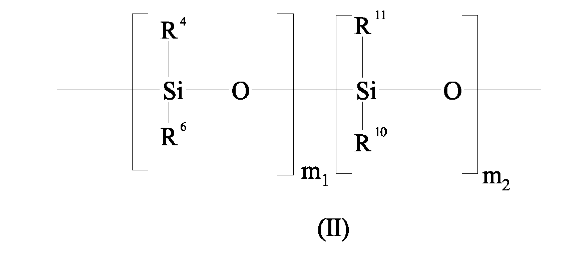 dans laquelle
   - R⁴ et R⁶, identiques ou différents, sont tels que définis ci-dessus pour la formule (I),
   - R¹⁰ représente un groupe tel que défini ci-dessus pour R⁴ et R⁶, ou représente le groupe de formule -X-G-R¹² dans laquelle X et G sont tels que définis ci-dessus pour la formule (I) et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
   - R¹¹ représente le groupe de formule -X-G-R¹² dans laquelle X, G et R¹² sont tels que définis ci-dessus,
   - m₁ est un nombre entier allant de 1 à 998, et
   - m₂ est un nombre entier allant de 2 à 500.

Selon l'invention, le polymère utilisé comme agent structurant, peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R⁴, R⁵, R⁶, R⁷, X, G, Y, m et n est différent dans l'un des motifs. Le copolymère peut être aussi formé de plusieurs motifs de formule (II), dans lequel l'un au moins des R⁴, R⁶, R¹⁰, R¹¹, m₁ et m₂ est différent dans l'un au moins des motifs.

On peut encore utiliser un copolymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des copolymères blocs, des copolymères séquencés ou des copolymères greffés.
1) Selon un premier mode de réalisation de l'invention, les groupes capables d'établir des interactions hydrogènes sont des groupes amides de formule - C(O)NH- et -HN-C(O)-.

Dans ce cas, l'agent structurant peut être un polymère comprenant au moins un motif de formule (III) ou (IV): 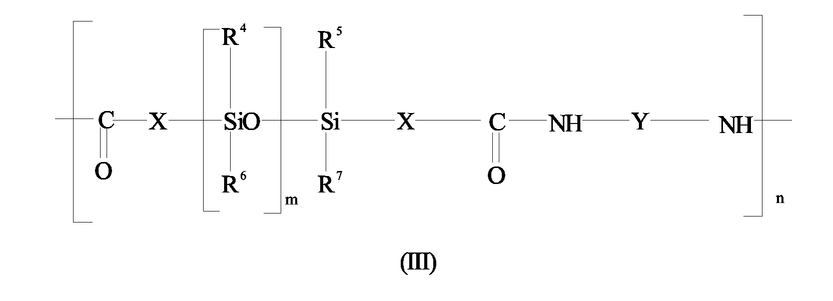 ou 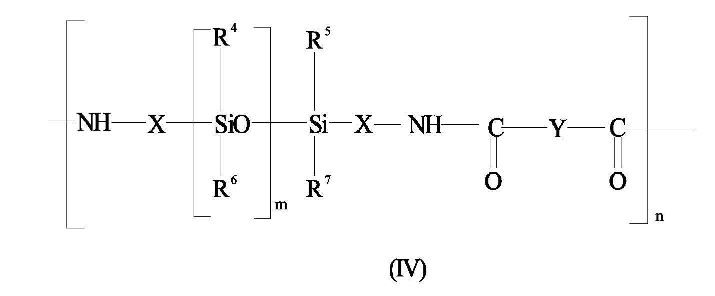 dans lesquelles R⁴, R⁵, R⁶, R⁷, X, Y, m et n sont tels que définis ci-dessus.

Un tel motif peut être obtenu :
- soit par une réaction de condensation entre un silicone à extrémités α, ω-acides carboxyliques et une ou plusieurs diamines, selon le schéma réactionnel suivant : 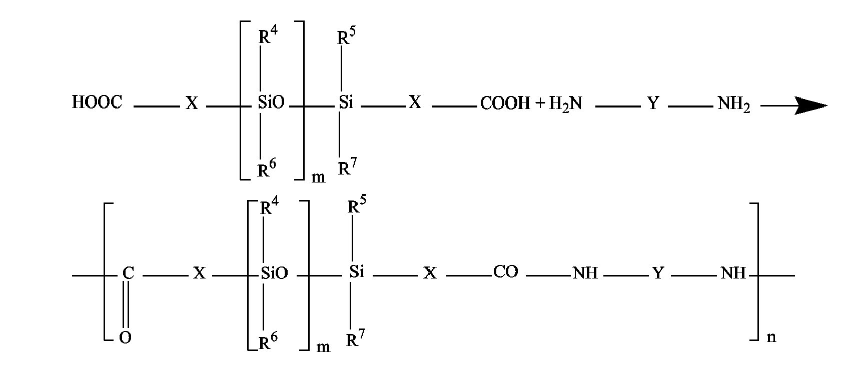
- soit par réaction de deux molécules d'acide carboxylique α-insaturé avec une diamine selon le schéma réactionnel suivant : 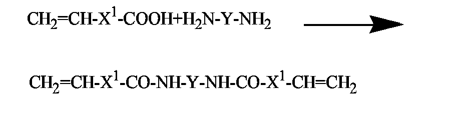 suivie de l'addition d'un siloxane sur les insaturations éthyléniques, selon le schéma suivant :

   CH₂=CH-X¹-CO-NH-Y-NH-CO-X¹-CH=CH₂ 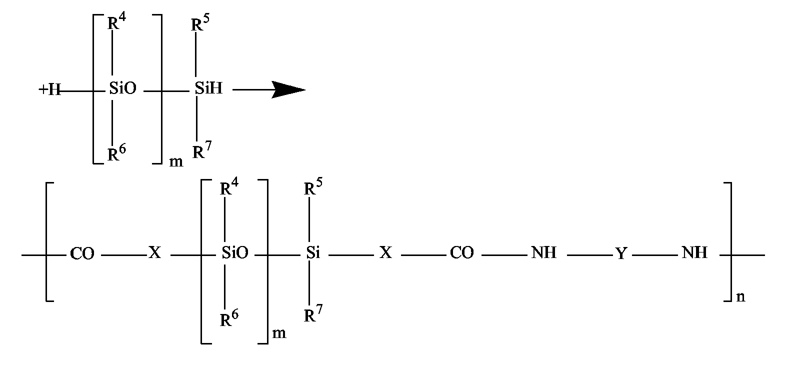
dans lesquels X¹-(CH₂)₂- correspond au X défini ci-dessus et Y, R⁴, R⁵, R⁶, R⁷ et m sont tels que définis ci-dessus,
- soit par réaction d'un silicone à extrémités α, ω-NH₂ et d'un diacide de formule HOOC-Y-COOH selon le schéma réactionnel suivant :

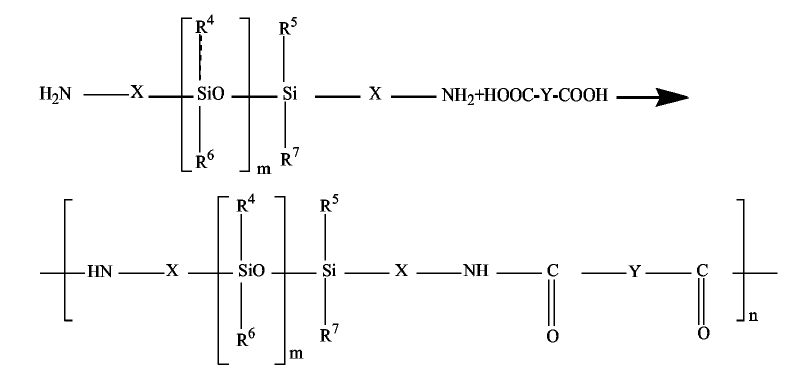

Dans ces polyamides de formule (III) ou (IV), m est de préférence dans la gamme de 1 à 700, en particulier de 15 à 500 et notamment de 50 à 200. En ce qui concerne n, il est en particulier dans la gamme de 1 à 500, de préférence de 1 à 100 et mieux encore de 4 à 25,
- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃.

Dans les formules (III) et (IV), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans ces formules (III) et (IV), Y peut aussi représenter : 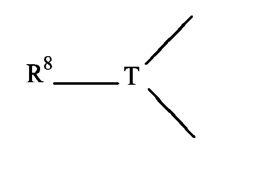 où R⁸ représente une chaîne polyorganosiloxane, et T représente un groupe de formule : 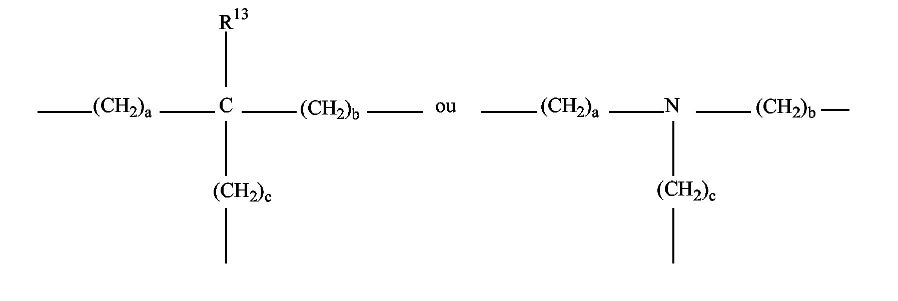 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷.

Dans les formules (III) et (IV), R⁴, R⁵, R⁶ et R⁷ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule (V): 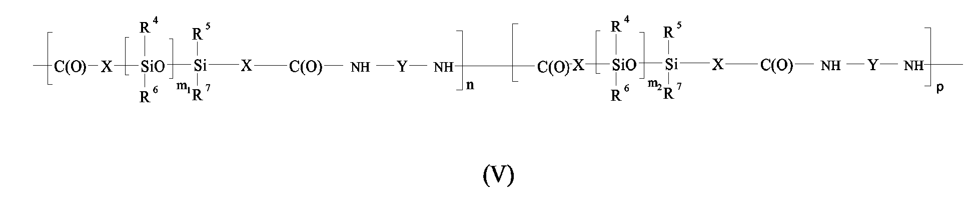 dans laquelle X, Y, n, R⁴ à R⁷ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la formule VI: 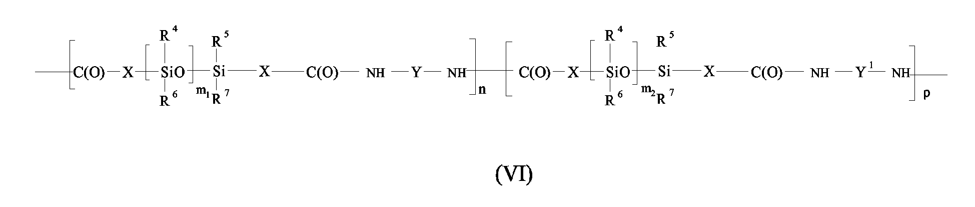 dans laquelle R⁴ à R⁷, X, Y, m₁, m₂, n et p ont les significations données ci-dessus et Y¹ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, l'agent structurant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif de formule (VII) : 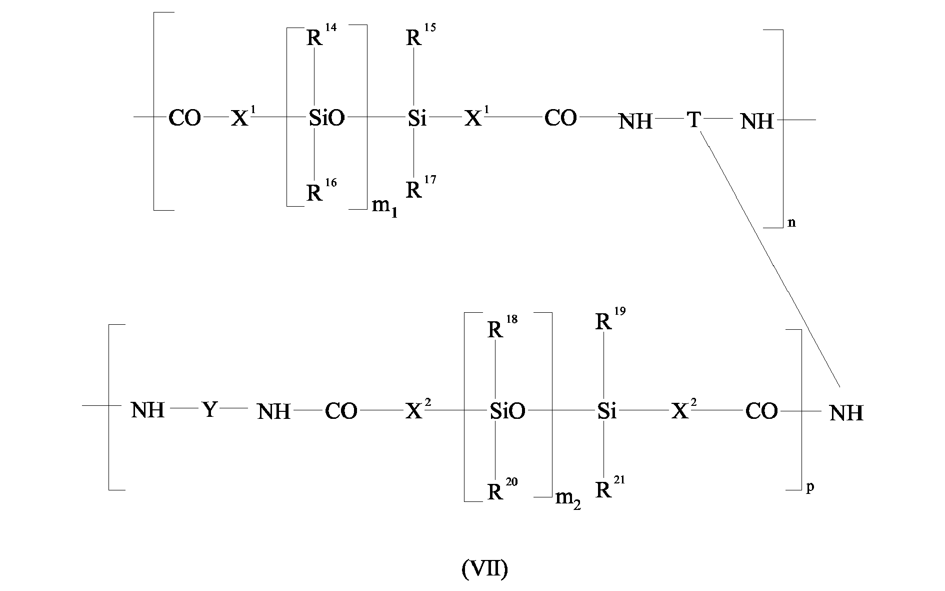 dans laquelle X¹ et X² qui sont identiques ou différents, ont la signification donnée pour X dans la formule (I), n est tel que défini dans la formule (I), Y et T sont tels que définis dans la formule (I), R¹⁴ à R²¹ sont des groupes choisis dans le même groupe que les R⁴ à R⁷, m₁ et m₂ sont des nombres situés dans la gamme allant de 1 à 1 000, et p est un nombre entier allant de 2 à 500.

Dans la formule (VII), on préfère que :
- p soit dans la gamme de 1 à 25, mieux encore de 1 à 7,
- R¹⁴ à R²¹ soient des groupes méthyle,
- T réponde à l'une des formules suivantes : 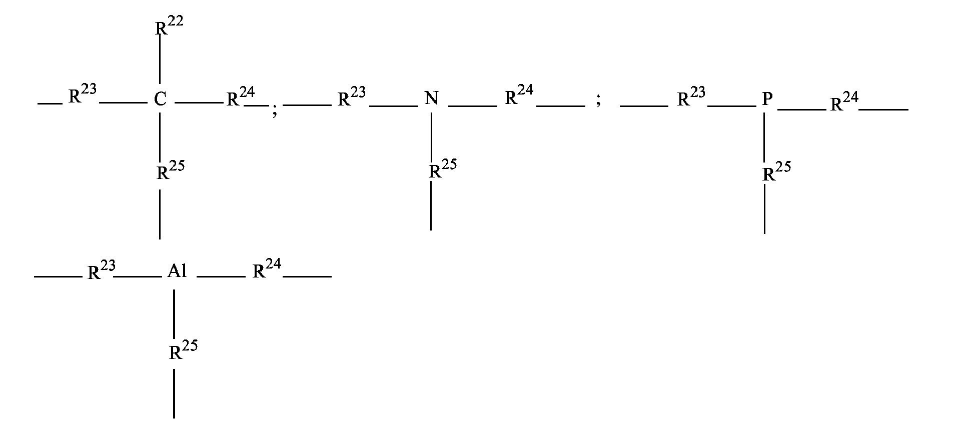
dans lesquelles R²² est un atome d'hydrogène ou un groupe choisi parmi les groupes définis pour R⁴ à R⁷, et R²³, R²⁴ et R²⁵ sont indépendamment des groupes alkylène, linéaires ou ramifiés, de préférence encore, à la formule : 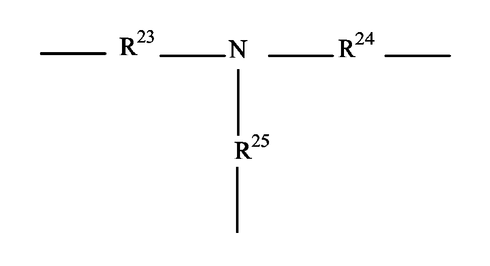 en particulier avec R²³, R²⁴ et R²⁵ représentant -CH₂-CH₂-,
- m₁ et m₂ soient dans la gamme de 15 à 500, et mieux encore de 15 à 45,
- X₁ et X₂ représentent -(CH₂)₁₀-, et
- Y représente -CH₂-.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids de motifs silicone greffé (VII) dans le copolymère peut aller de 0,5 à 30 % en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon l'invention, les polyamides à base de siloxanes préférés sont :
- les polyamides de formule (III) où m varie de 50 à 600, en particulier de 60 à 400, notamment de 75 à 200 et plus particulièrement est de l'ordre de 100 ;
- des mélanges de polyamide de formule (III) combinant :
   1) 80 à 99 % en poids d'un polyamide où m varie de 50 à 600, en particulier de 60 à 400, notamment de 75 à 200 et plus particulièrement est de l'ordre de 100 et
   2) 1 à 20 % d'un polyamide où m est dans la gamme de 5 à 100, en particulier de 10 à 75 et plus particulièrement est de l'ordre de 15;
- des mélanges de polyamide de formule (III) combinant :
   1) 80 à 99 % en poids d'un polyamide où n varie de 2 à 10, en particulier de 3 à 6, et
   2) 1 à 20 % d'un polyamide où n est dans la gamme de 5 à 500, en particulier de 30 à 100 ;
- des mélanges de polyamide de formule (III) combinant :
   1) 1 à 20 % en poids d'un polyamide, où n est égal à 2 à 10, en particulier de 3 à 6, et
   2) 80 à 99 % d'un polyamide, où n est dans la gamme de 30 à 500, en particulier de 30 à 100 ;
- des polyamides de formule (III) où X représente un radical alkyle en C₃ à C₁₅ en particulier en C₅ à C₁₂ et notamment en C₁₀ et
- des polyamides de formule (III) où Y représente un radical alkyle en C₃ à C₁₀ en particulier en C₄ à C₈ et notamment en C₆.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Avantageusement, la composition selon l'invention comprend au moins un copolymère bloc polydiméthylsiloxane de formule générale (I) possédant un indice m de valeur supérieure à 50, en particulier supérieure à 75 et notamment d'environ 100.

Selon un mode de réalisation particulier, une telle composition comprend en outre au moins un copolymère bloc polydiméthylsiloxane de formule générale (I) possédant un indice m de valeur 15.

Des copolymères texturant à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamine et/ou des oligosiloxanes-diamines (réaction d'amidation), ou alternativement avec des oligosiloxanes alcools ou des oligosiloxanes diols (réaction d'estérification). La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonés, des siloxanes diamines avec 1 à 300, plus particulièrement 2 à 50, et mieux encore 2, 6, 9, 5, 12, 13,5, 23 ou 31 groupes siloxanes, peuvent être utilisés pour la réaction avec des polyamides hydrocarbonées à base de dimères d'acide gras.

Les réactions peuvent être effectuées dans le xylène pour extraire l'eau produite de la solution par distillation azéotropique, ou à des températures plus élevées (autour de 180 à 200 °C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1 % en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

On peut aussi préparer un copolymère de polyamide-silicone, en utilisant un polyamide à groupes amines libres, par réaction d'amidation avec un siloxane contenant un groupe acide.

On peut encore préparer un copolymère texturant à base de copolymère entre un polyamide hydrocarboné et un polyamide siliconé, par transamidation d'un polyamide ayant par exemple un constituant éthylène-diamine, par une oligosiloxane-α, ω-diamine, à température élevée (par exemple 200 à 300 °C), pour effectuer une transamidation de sorte que le composant éthylène diamine du polyamide d'origine est remplacé par l'oligosiloxane diamine.

Le copolymère de polyamide hydrocarboné et de polyamide-silicone peut encore être un copolymère greffé comportant un squelette de polyamide hydrocarboné avec des groupes oligosiloxane pendants.

Ceci peut être obtenu par exemple :
- par hydrosilylation de liaisons insaturées dans des polyamides à base de dimères d'acides gras,
- par silylation des groupes amides d'un polyamide, ou
- par silylation de polyamides insaturés au moyen d'une oxydation, c'est-à-dire en oxydant les groupes insaturés en alcools ou diols, pour former des groupes hydroxyle que l'on fait réagir avec des acides siloxane carboxyliques ou des siloxanes-alcools. On peut aussi époxyder les sites oléfiniques des polyamides insaturés puis faire réagir les groupes époxy avec des siloxanes-amines ou des siloxanes-alcools.

2) Selon un second mode de réalisation de l'invention, le copolymère texturant est constitué par un homopolymère ou copolymère comportant des groupes uréthane ou urée.

Comme précédemment, le polymère peut comporter des motifs polyorganosiloxanes contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante (VIII) : 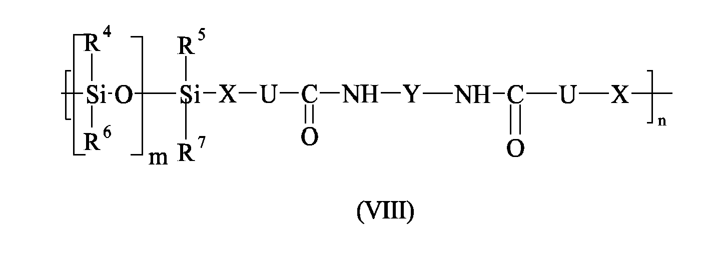 dans laquelle les R⁴, R⁵, R⁶, R⁷, X, Y, m et n ont les significations données ci-dessus pour la formule (I), et U représente -O- ou -NH-, afin que : 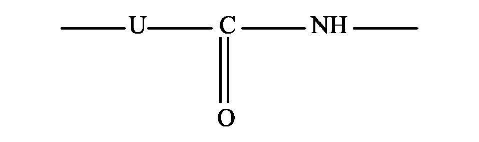 corresponde à un groupe uréthane ou urée.

Dans cette formule (VIII), Y peut être un groupe alkylène, en C₁ à C₄₀, linéaire ou ramifié, substitué éventuellement par un groupe alkyle en C₁ à C₁₅ ou un groupe aryle en C₅ à C₁₀. De préférence, on utilise un groupe -(CH₂)₆-.

Y peut aussi représenter un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en C₁ à C₄₀, linéaire ou ramifié, ou un radical cycloalkylène en C₄ à C₁₂.

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane ou urée dans la chaîne alkylène.

Il peut répondre à la formule (IX) : 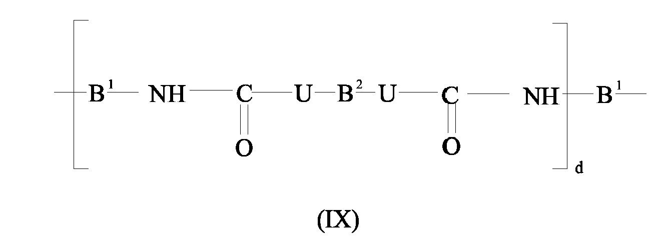 dans laquelle B¹ est un groupe choisi parmi les groupes donnés ci-dessus pour Y, U est -O-
ou -NH-, et B² est choisi parmi :
- les groupes alkylène en C₁ à C₄₀, linéaires ou ramifiés,
- les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en C₁ à C₃, et
- les groupes de formule : 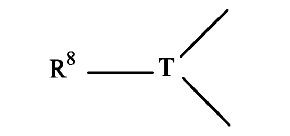
dans laquelle T est un radical trivalent hydrocarboné pouvant contenir un ou plusieurs hétéroatomes tels que l'oxygène, le soufre et l'azote et R⁸ est une chaîne polyorganosiloxane ou une chaîne alkyle en C₁ à C₅₀, linéaire ou ramifiée.

T peut représenter par exemple : 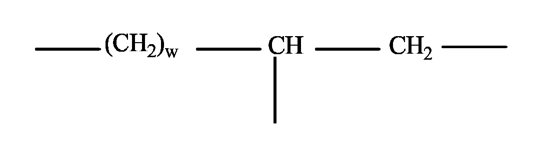 ou 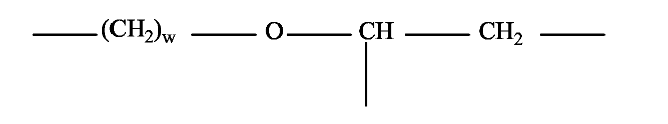 avec w étant un nombre entier allant de 1 à 10 et R⁸ étant une chaîne polyorganosiloxane.

Lorsque Y est un groupe alkylène, en C₁ en C₄₀ linéaire ou ramifié, on préfère les groupes -(CH₂)₂- et -(CH₂)₆-.

Dans la formule donnée ci-dessus pour Y, d peut être un entier allant de 0 à 5, de préférence de 0 à 3, de préférence encore égal à 1 ou 2.

De préférence B² est un groupe alkylène en C₁ à C₄₀, linéaire ou ramifié, en particulier -(CH₂)₂- ou -(CN₂)₆-, ou le groupe : 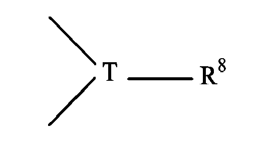 avec R⁸ étant une chaîne polyorganosiloxane.

Comme précédemment, le polymère constituant le copolymère texturant peut être formé de motifs silicone uréthane et/ou silicone-urée de longueur et/ou de constitution différentes, et se présenter sous la forme de copolymères blocs, séquencés ou statistiques (aléatoires).

Les polymères de formule (VIII) comportant des groupes urées ou uréthanes dans la chaîne du polymère siliconé peuvent être obtenus par réaction entre un silicone à groupes terminaux α,ω-NH₂ ou -OH, de formule : 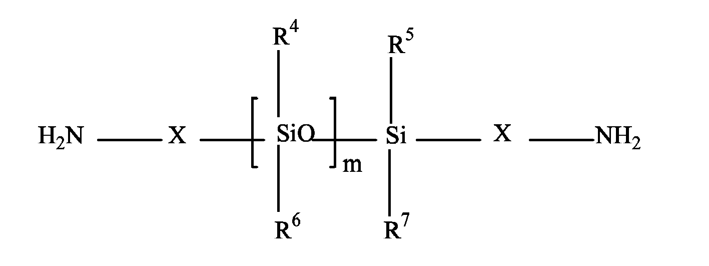 dans laquelle m, R⁴, R⁵, R⁶, R⁷ et X sont tels que définis pour la formule (I), et un diisocyanate OCN-Y-NCO où Y a la signification donnée dans la formule (I) ; et éventuellement un coupleur diol ou diamine de formule H₂N-B²-NH₂ ou HO-B²-OH, où B² est tel que défini dans la formule (IX).

Suivant les proportions stoechiométriques entre les deux réactifs, diisocyanate et coupleur, on pourra avoir pour Y la formule (IX) avec d égale 0 où d égale 1 à 5.

Comme dans le cas des polyamides silicones de formule (IV), (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule : 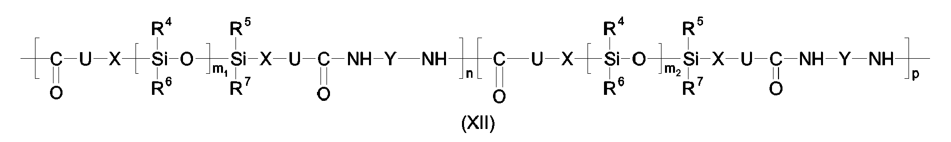 dans laquelle R⁴, R⁵, R⁶, R⁷, X, Y et U sont tels que définis pour la formule (VIII) et m₁, m₂, n et p sont tels que définis pour la formule (V).

Selon l'invention, le silicone peut aussi comporter les groupes uréthane et/ou urée non plus dans le squelette mais en ramifications latérales.

Dans ce cas, le polymère peut comprendre au moins un motif de formule : 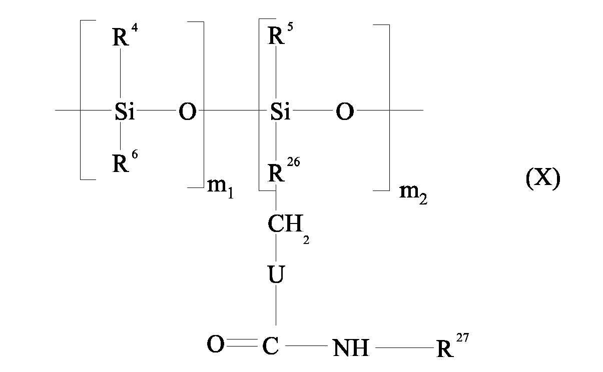 dans laquelle R⁴, R⁶, R⁵, m₁ et m₂ ont les significations données ci-dessus pour la formule (II), et R⁵ pour la formule (I),
- U représente O ou NH,
- R²⁶ représente un groupe alkylène en C₁ à C₄₀, comportant éventuellement un ou plusieurs hétéroatomes choisis parmi O et N, ou un groupe phénylène, et
- R²⁷ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

Les polymères comportant au moins un motif de formule (X) contiennent des unités siloxanes et des groupes urées ou uréthanes, et ils peuvent être utilisés comme copolymère texturant dans les compositions de l'invention.

Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.

Ils peuvent être obtenus à partir de polysiloxanes ramifiés, comportant un ou deux groupes amino par ramification, en faisant réagir ces polysiloxanes avec des monoisocyanates.

A titre d'exemples de polymères de départ de ce type ayant des ramifications amino et diamino, on peut citer les polymères répondant aux formules suivantes :

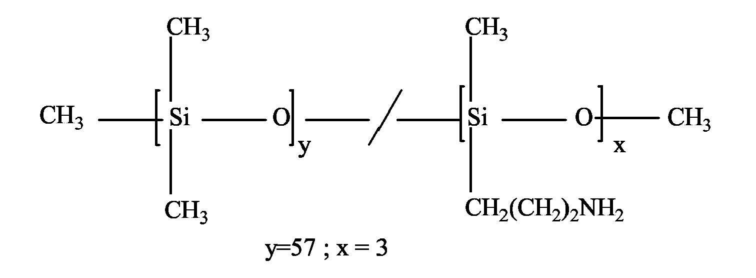

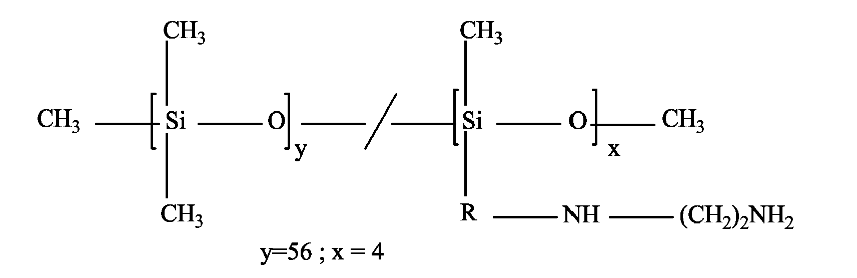

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.

De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales, ou bien les groupes amino peuvent être aux extrémités de la chaîne principale de sorte que les groupes capables d'interaction hydrogène seront des groupes terminaux du polymère.

Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.

Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.

A titre d'illustration d'un polysiloxane terminé par quatre groupes urées, on peut citer le polymère de formule : 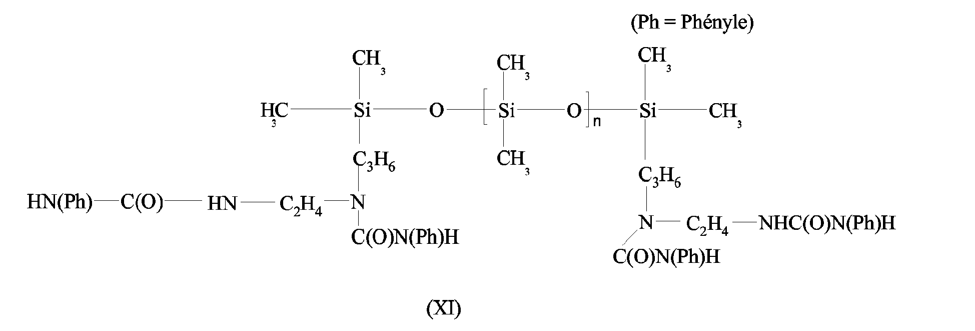 où Ph est un groupe phényle et n est un nombre de 0 à 300, en particulier de 0 à 100, par exemple de 50.

Ce polymère est obtenu par réaction du polysiloxane à groupes amino suivant : 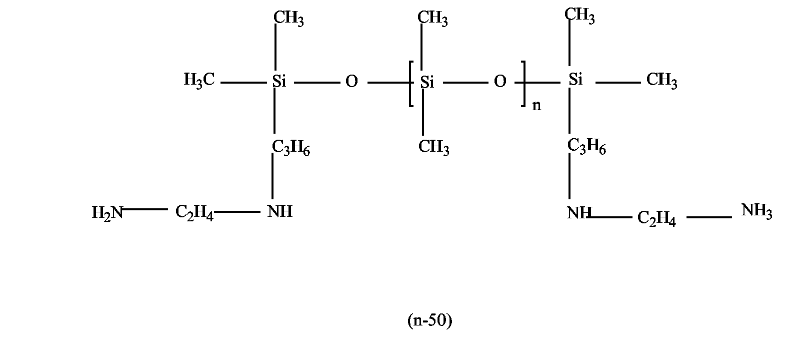 avec l'isocyanate de phényle.

On peut obtenir également des polyuréthanes ou polyurées silicones ramifiés en utilisant à la place du diisocyanate OCN-Y-NCO, un triisocyanate de formule :

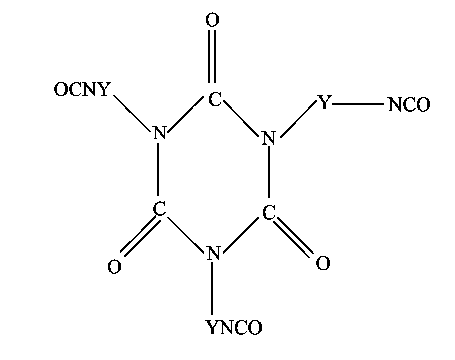

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule : 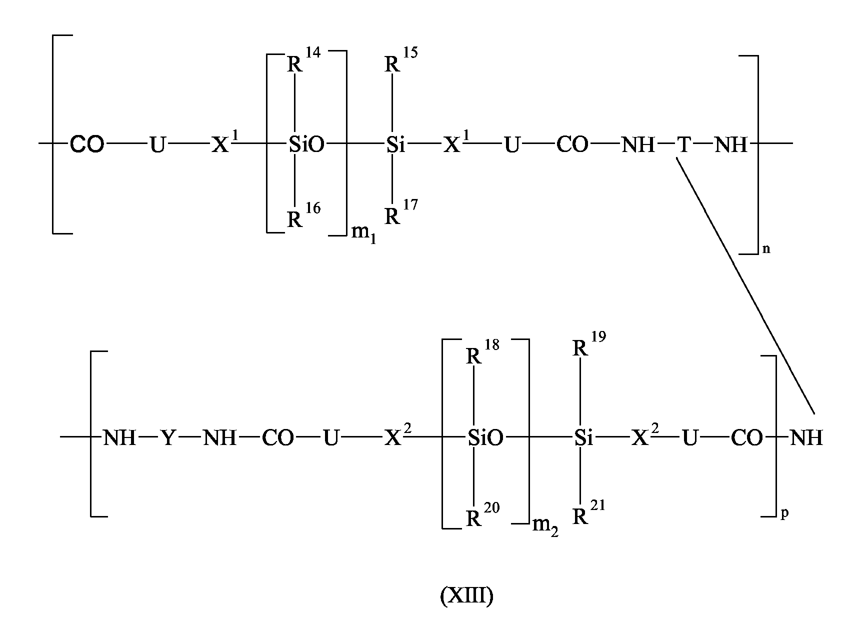 dans laquelle X¹ et X² qui sont identiques ou différents, ont la signification donnée pour X dans la formule (I), n est tel que défini dans la formule (I), Y et T sont tels que définis dans la formule (I), R¹⁴ à R²¹ sont des groupes choisis dans le même groupe que les R⁴ à R⁷, m₁ et m₂ sont des nombres situés dans la gamme allant de 1 à 1 000, et p est un nombre entier allant de 2 à 500.

Comme dans le cas des polyamides, ce copolymère peut comporter aussi des motifs polyuréthanes silicones sans ramification.

Les polyurées et les polyuréthanes à base de siloxanes préférés sont :
- les polymères de formule (VIII) où m varie de 50 à 600, en particulier de 60 à 400, notamment de 75 à 200 et plus particulièrement de l'ordre de 100 ;
- des mélanges de polymères de formule (VIII) combinant :
   1) 80 à 99 % en poids d'un polymère de formule (VIII) où m varie de 50 à 600, en particulier de 60 à 400, notamment de 75 à 200 et plus particulièrement de l'ordre de 100, et
   2) 1 à 20 % d'un polymère de formule (VIII) où m est dans la gamme de 5 à 100, en particulier de 10 à 75 et plus particulièrement est de l'ordre de 15;
- des mélanges de polymères de formule (VIII) combinant :
   1) 80 à 99 % en poids de polymères de formule (VIII) où n est égal à 2 à 10, en particulier 3 à 6, et
   2) 1 à 20 % de polymères de formule (VIII) où n est dans la gamme de 5 à 500, en particulier de 30 à 100 ;
- des mélanges de polymères de formule (VIII) combinant :
   1) 1 à 20 % en poids de polymères de formule (VIII), où n est égal à 2 à 10, en particulier 3 à 6, et
   2) 80 à 99 % de polymères de formule (VIII), où n est dans la gamme de 30 à 500, en particulier de 30 à 100 ;
- des polymères de formule (VIII) où X représente un radical alkyle en C₃ à C₁₅ en particulier en C₅ à C₁₂ et notamment en C₁₀ et
- des polymères de formule (VIII) où Y représente un radical alkyle en C₃ à C₁₀ en particulier en C₄ à C₈ et notamment en C₆.

Comme dans le cas des polyamides, on peut utiliser dans l'invention des copolymères de polyuréthane -ou de polyurée- silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les copolymères de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales ou ramifications de la chaîne principale. Ceci peut correspondre aux cinq dispositions suivantes : 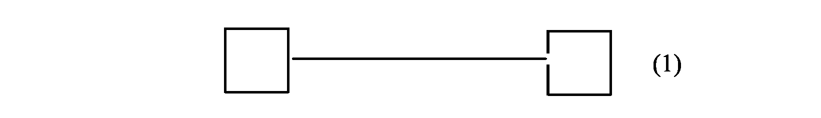 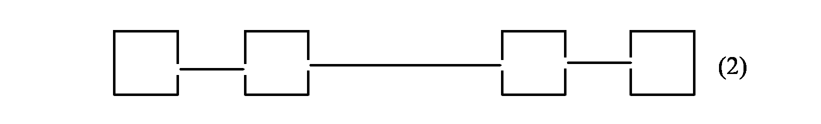 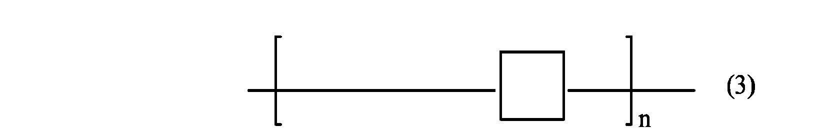 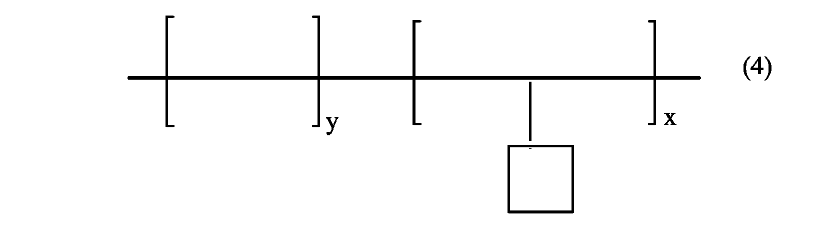 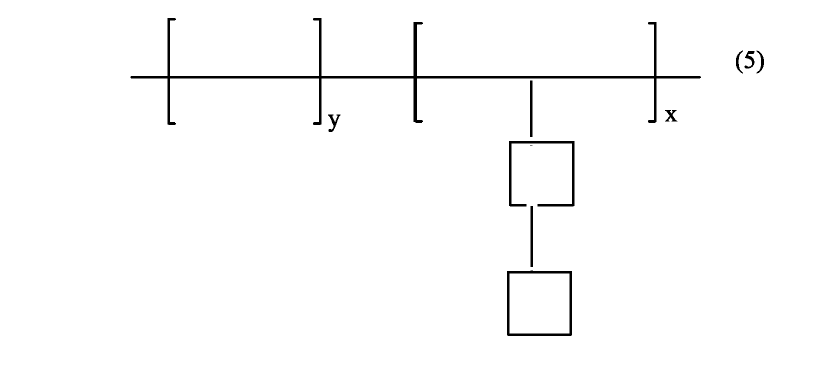 dans lesquelles, la ligne continue est la chaîne principale du polymère siloxane et les carrés représentent les groupes capables d'établir des interactions hydrogène.

Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène. Les valeurs n, x et y sont telles que le polymère présente les propriétés voulues en tant qu'agent structurant de phases grasses à base d'huile siliconée.

Selon une variante de l'invention, la texture de la phase grasse liquide contenant au moins une huile est obtenue à l'aide d'un ou plusieurs des polymères mentionnés ci-dessus.

A titre d'exemples de polymères utilisables, on peut citer les polyamides siliconés, obtenus conformément aux exemples 1 à 3 du document US-A-5 981 680.

Ils présentent une bonne solubilité dans les huiles siliconées et les huiles esters et conduisent à des compositions macroscopiquement homogènes. De préférence, ils ont une masse moléculaire moyenne de 500 à 200 000, par exemple de 10 000 à 150 000, de préférence de 20 000 à 100 000.

Le ou les copolymère(s) siliconé(s) peut(vent) représenter de 0,5 à 30 % en poids, notamment de 1 à 30 % en poids et plus particulièrement 5 à 30 % en poids de la composition.

### COPOLYMERE ORGANIQUE NON SILICONE

Selon une seconde variante de l'invention, le copolymère organique est non siliconé.

Plus précisément, il s'agit de polymères et plus particulièrement de copolymères résultant de la copolymérisation d'au moins un monomère éthylénique, en particulier on peut utiliser des copolymères vinyliques, acryliques ou méthacryliques. Ce composé peut comprendre, par exemple, un bloc styrène (S) ou un bloc alkylstyrène (AS), et un bloc choisi parmi les blocs éthylène/butylène (EB), éthylène/propylène (EP), butadiène (B), isoprène (I), acrylate (A), méthacrylate (MA) ou une association de ces blocs.

Dans un mode particulier de réalisation, un copolymère comprenant au moins un bloc styrène est utilisé. Un copolymère tribloc et en particulier ceux du type polystyrène/polyisoprène ou polystyrène/polybutadiène, tels que ceux commercialisés ou fabriqués sous la dénomination "LUVITOL HSB" par BASF et ceux du type polystyrène/copoly(éthylène-propylène) ou de manière alternative ceux du type polystyrène/copoly(éthylène/butylène), tels que ceux commercialisés ou fabriqués sous la marque de fabrique "KRATON" par SHELL CHEMICAL Co. ou GELLED PERMETHYL 99A par PENRECO, peuvent être utilisés. Des copolymères de styrène-méthacrylate peuvent également être utilisés.

Comme gélifiant éthylénique susceptible d'être utilisé dans les compositions conformes à l'invention, on peut citer par exemple, le KRATON G1650 (SEBS), le KRATON G1651 (SEBS), le KRATON G1652 (SEBS), le KRATON G1657X (SEBS), le KRATON G1701X (SEP), le KRATON G1702X (SEP), le KRATON G1726X (SEB), le KRATON D-1101 (SBS), le KRATON D-1102 (SBS), le KRATON D-1107 (SIS), le GELLED PERMETHYL 99A-750, le GELLED PERMETHYL 99A-753-58, le GELLED PERMETHYL 99A-753-59, le VERSAGEL 5970 et le VERSAGEL 5960 de chez PENRECO, et OS 129880, OS 129881 et OS 84383 de chez LUBRIZOL (copolymère de styrène-méthacrylate).

Des di- ou triblocs tels que le polystyrène-copoly(éthylène/propylène) ou le polystyrène-copoly(éthylène/butylène) tels que ceux décrits dans les demandes de brevet WO 98/38981 et US 2002/0055562 sont également inclus dans la présente invention.

### PHASE GRASSE LIQUIDE

Les compositions cosmétiques conformes à la présente invention comprennent une phase grasse liquide à base d'au moins une huile.

### a. Huile siliconée

Selon une variante de l'invention, la phase grasse liquide comprend au moins une huile siliconée volatile.

L'huile siliconée volatile peut être choisie parmi les huiles siliconées linéaires ou cycliques ayant un point éclair égal ou supérieur à 40°C et avantageusement supérieur à la température de ramollissement du polymère structurant et/ou une viscosité inférieure à 8 cSt, telles que les polydiméthylsiloxanes (PDMS) linéaires ou cycliques ayant de 3 à 7 atomes de silicium.

Le point éclair est la température à laquelle un carburant prend feu au contact d'une flamme.

A titre d'exemple de telles huiles, on peut citer les composés cités dans le tableau 1 ci-dessous.

Avantageusement, l'huile volatile présente un point éclair supérieur à 60°C.

Les huiles siliconées non volatiles peuvent être des polydiméthylsiloxanes, des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.

Les huiles siliconées non volatiles utilisables dans la phase grasse liquide de l'invention, qui contient au moins une huile ester, peuvent être en particulier les polydiméthylsiloxanes (PDMS) non volatils, linéaires, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et leurs mélanges.

La phase grasse liquide peut comprendre, outre l'huile ester essentielle selon l'invention, au moins une huile siliconée volatile et au moins une huile non siliconée volatile.

Au sens de l'invention, une huile volatile siliconée ou non siliconée présente un point éclair de préférence de 40 à 135°C ou pas de point éclair. Les huiles volatiles présentent à température ambiante (25°C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa).

Les huiles siliconées ont une viscosité choisie avantageusement dans la gamme allant de 5 à 800 000 cSt à 25°C, de préférence de 10 à 500 000 cSt, et mieux de 10 à 5 000 cSt.

**Tableau 1**

| Composé | Point éclair (°C) | Viscosité (cSt) |
|---|---|---|
| Octyltriméthicone | 93 | 1,2 |
| Hexyltriméthicone | 79 | 1,2 |
| Décaméthyl cyclopentasiloxane (cyclopentasiloxane ou D5) | 72 | 4,2 |
| Octaméthylcyclo tétrasiloxane (cyclotétradiméthyl siloxane ou D4) | 55 | 2,5 |
| Dodécaméthylcyclo hexasiloxane (D6) | 93 | 7 |
| Décaméthyltétrasiloxane (L4) | 63 | 1,7 |
| KF 96 A de Shin Etsu | 94 | 6 |
| PDMS (polydiméthylsiloxane) DC 200 (1,5 cSt) de Dow Corning | 56 | 1,5 |
| PDMS DC 200 (2 cSt) de Dow Corning | 87 | 2 |
| PDMS DC 200 (5 cSt) de Dow Corning | 134 | 5 |
| PDMS DC 200 (3 cSt) de Dow Corning | 102 | 3 |

En d'autres termes, la(les) huile(s) siliconée(s) volatile(s) peut(vent) être choisie(s), par exemple, dans le groupe constitué par les composés du tableau 1, l'heptaméthyloctyltrisiloxane, le dodécaméthylpentasiloxane et leurs mélanges.

L'huile siliconée volatile peut aussi être choisie dans le groupe des huiles siliconées fluorées telles que les silicones à groupes alkyle et perfluoralkyle, les silicones à groupes latéraux oxyéthylénés/oxypropylénés (OE/PP) et à groupes perfluorés, les silicones à groupes latéraux perfluorés et à groupes latéraux glycérolés, et les perfluoroalkylméthylphénylsiloxanes, ces huiles ayant une pression de vapeur supérieure ou égale à 0,02 mm Hg.

La phase grasse liquide contient avantageusement au moins 5 %, par exemple de 10 à 90 % en poids d'huile(s) siliconée(s). La phase grasse liquide contient avantageusement au moins une huile siliconée qui a avantageusement une viscosité inférieure à 1 000 cSt et mieux inférieure à 100 cSt.

### b. Huile ester

Selon une variante de l'invention, au moins une des huiles de la phase grasse liquide est une huile, dite "huile ester", qui est choisie parmi les esters des acides monocarboxyliques avec les monoalcools et polyalcools.

Avantageusement, ledit ester répond à la formule suivante :

R₁-CO-O-R₂

où R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par "éventuellement substitué", on entend que R₁ et ou R₂ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de R₁ + R₂ est ≥ 9.

L'ester, mis en oeuvre selon l'invention, peut être désigné sous le nom d'ester "court".

R₁ peut représenter le reste d'un acide gras, de préférence supérieur, linéaire ou de préférence ramifié comprenant de 1 à 40 et mieux de 7 à 19 atomes de carbone et R₂ peut représenter une chaîne hydrocarbonée linéaire ou de préférence ramifiée contenant de 1 à 40, de préférence de 3 à 30 et mieux de 3 à 20, (19 à 28, 8 à 27, 7 à 26 C) atomes de carbone. De nouveau, de préférence, le nombre d'atomes de carbone de R₁ + R₂ ≥ 9.

Des exemples des groupes R₁ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

Des exemples d'esters utilisables dans les phases grasses des compositions de l'invention sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (I) ci-dessus, dans laquelle R₁ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et R₂ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone. De préférence dans la formule (I), R₁-CO- et R₂ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :
- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle.

L'ester préféré entre tous est l'isononanoate d'isononyle qui permet d'obtenir au mieux des compositions présentant une excellente transparence, translucidité alliée à d'excellentes propriétés de non-transfert et d'absence de collant.

Avantageusement, la phase grasse liquide comprend de 0,5 à 100 % en poids de l'huile ou des huiles esters, de préférence de 1 à 80 % en poids, de préférence encore de 2 à 50 % poids, mieux de 2 à 40 % en poids.

Plus particulièrement, la phase grasse comprend au moins l'isononanoate d'isononyle à titre d'huile ester.

### c. Huile non siliconée

La phase grasse liquide des compositions selon l'invention peut contenir en outre une ou plusieurs huiles non siliconées volatiles ou non. Les huiles non siliconées volatiles peuvent être choisies dans le groupe des huiles hydrocarbonées et des esters et éthers volatils tels que les hydrocarbures volatils comme l'isododécane et l'isohexadécane, les isoparaffines en C₈-C₁₆.

L'huile non siliconée volatile peut aussi être choisie parmi les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.

A titre d'exemple d'huiles non siliconées volatiles utilisables dans la composition de l'invention, on peut citer les composés du tableau 2 qui suit.

**Tableau 2**

| Composé | Point éclair (°C) |
|---|---|
| Isododécane | 43 |
| Isohexadécane | 102 |
| n-butyléther de propylène glycol | 60 |
| 3-éthoxypropionate d'éthyle | 58 |
| Acétate de méthyléther de propylène glycol | 46 |
| Isopar L (isoparaffine C11-C13) | 62 |
| Isopar H (isoparaffine C11-C12) | 56 |

Lorsque la phase grasse comprend une huile volatile, celle-ci représente avantageusement de 3 à 89,4 %, et mieux de 5 à 60 %, par exemple de 5 à 20 % du poids total de la composition.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse ayant de 10 à 40 atomes de carbone,
- les alcools gras en C₈ à C₂₆ comme l'alcool oléique ou l'octyldodécanol,
- les acides gras comme l'acide oléique, linoléique ou linolénique, et
- leurs mélanges.

La phase grasse liquide peut encore contenir des huiles apolaires telles que les hydrocarbures ou fluorocarbures, linéaires ou ramifiés, d'origine synthétique ou minérale, volatils ou non, comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges.

Ainsi, l'invention peut être mise en oeuvre, par exemple, avec les différentes phases grasses suivantes :
1) une phase grasse constituée d'un mélange d'huiles comprenant au moins une huile ester et au moins une huile siliconée volatile ;
2) une phase grasse constituée d'un mélange d'huiles comprenant au moins une huile ester et au moins une huile volatile non siliconée ;
3) une phase grasse constituée d'un mélange d'huiles comprenant au moins une huile ester, au moins une huile non siliconée volatile et éventuellement une huile siliconée volatile ;
4) une phase grasse constituée d'un mélange d'huiles comprenant au moins une huile ester, une huile non siliconée, non volatile et éventuellement au moins une huile non siliconée volatile ; et
5) une phase grasse telle que définie en 1) à 4) sans huile ester.

Généralement, la phase grasse liquide représente de 5 à 95 % du poids total de la composition et mieux de 20 à 75 %.

### AUTRES CORPS GRAS

Les compositions selon l'invention peuvent en outre comprendre au moins un corps gras solide, qui peut être choisi parmi les cires et les composés pâteux.

Plus particulièrement, les compositions selon l'invention peuvent comprendre de 0,1 % à 40 % en poids, notamment de 0,1 % à 30 % en poids, et plus particulièrement de 0,5 % à 25 % en poids de corps gras solide(s) par rapport au poids total de la composition.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

La cire peut également présenter une dureté allant de 0,05 MPa à 15 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées, fluorées et/ou siliconées et être d'origine végétale, minérale, animale et/ou synthétique. En particulier, les cires présentent une température de fusion supérieure à 30 °C et mieux supérieure à 45 °C.

Comme cire utilisable dans la première composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les compositions peuvent également contenir une cire micronisée, appelée également micro cire.

Comme micro cires pouvant être utilisées dans les compositions selon l'invention, on peut citer notamment les micro cires de carnauba telles que celle commercialisée sous la dénomination de "MicroCare 350® " par la société MICRO POWDERS, les micro cires de cire synthétique telles que celle commercialisée sous la dénomination de "MicroEase 114S®" par la société MICRO POWDERS, les micro cires constituées d'un mélange de cire de carnauba et de cire de polyéthylène telles que celles commercialisées sous les dénominations de "Micro Care 300®" et "310®" par la société MICRO POWDERS, les micro cires constituées d'un mélange de cire de carnauba et de cire synthétique telles que celle commercialisée sous la dénomination "Micro Care 325®" par la société MICRO POWDERS, les micro cires de polyéthylène telles que celles commercialisées sous les dénominations de "Micropoly 200®", "220®", "220L®" et "250S®" par la société MICRO POWDERS et les micro cires de polytétrafluoroéthylène telles que celles commercialisées sous les dénominations de "Microslip 519®" et "519 L®" par la société MICRO POWDERS.

Parmi les micro cires citées précédemment, certaines telles que par exemple la micro cire de carnauba, la micro cire de cire synthétique "MicroEase 114S», ou la micro cire constituée d'un mélange de cire de carnauba et de cire synthétique "MicroCare 325" présentent un point de fusion commençante supérieur ou égal à 45 °C.

A titre indicatif, les compositions peuvent contenir de 0,1 à 50 % en poids et mieux de 1 à 30 % en poids de cire par rapport à leur poids total.

Ces compositions peuvent en outre contenir au moins un composé pâteux, qui peut être avantageusement choisi parmi
- la lanoline et ses dérivés
- les composés siliconés polymères ou non
- les composés fluorés polymères ou non
- les polymères vinyliques, notamment :
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- et leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ®, et Risocast DA-L ®,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

La composition peut le cas échéant comprendre au moins une phase aqueuse qui peut ou non être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄ et les aldéhydes en C₂₋C₄.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids, par rapport au poids total de la composition.

### TENSIOACTIF

Les compositions selon l'invention peuvent en outre contenir un ou plusieurs tensioactifs.

Selon une variante de l'invention, elles comprennent au moins un tensioactif siliconé.

Le ou les tensio-actifs siliconé(s) peut être présent dans la composition en une teneur allant de 0,1 % à 50 % en poids, par rapport au poids total de la composition, en particulier allant de 0,1 % à 40 % en poids, plus particulièrement allant de 0,5 % à 30 % en poids, en particulier allant de 0,5 % à 20 % en poids, et encore plus particulièrement allant de 1 % à 10 % en poids.

Parmi les agents tensioactifs utilisables dans les compositions cosmétiques conformes à la présente invention, on peut mentionner plus particulièrement les organopolysiloxanes hydrophiles différents du polymère siliconé décrit précédemment.

Le radical hydrophile peut répondre à la formule : 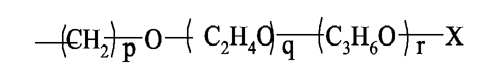 dans laquelle
- p varie de 0 à 5, q varie de 0 à 100, r varie de 0 à 50, avec p ou q étant différent de zéro,
- les unités (C₂H₄ O) et (C₃ H₆ O) peuvent être réparties aléatoirement ou par blocs, et
- X est un hydrogène ou un radical alkyle en C₁-C₁₀ le cas échéant substitué par un ou plusieurs fonctions de type hydroxyle, thiol, amine, carboxylique, carboxylate, amide, phosphate, sulfate et sulfonate.
   En particulier, p peut varier de 1 à 5, q de 1 à 100 et r de 1 à 50. X peut plus particulièrement figurer un atome d'hydrogène.
   En particulier, l'organopolysiloxane, selon l'invention, peut comprendre à titre de radical hydrophile au moins un radical hydroxy-polyalkylèneoxy et notamment un radical hydroxy-polyéthylèneoxy.
   Notamment, l'organopolysiloxane selon l'invention peut répondre à la formule : 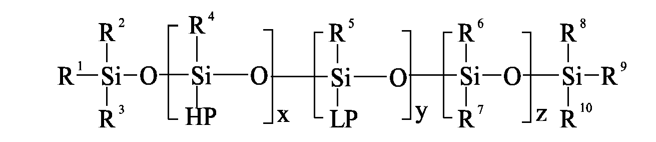
dans laquelle
- R¹, R², R³, R⁴, R⁵, R⁶ R⁷, R⁸, R⁹ et R¹⁰ représentent indépendamment l'un de l'autre un radical alkyle en C₁ à C₆, linéaire, ramifié ou cyclique, saturé ou non,
- HP est un radical portant au moins un groupement hydrophile tel que défini précédemment,
- LP est un radical lipophile, et
- x varie de 1 à 5000 ; y de 0 à 5000 ; z de 0 à 5000.

En ce qui concerne le radical LP, il peut notamment être choisi parmi des alkyles en C₁ à C₄₀ ramifiés, cycliques ou linéaires, des groupements organosiloxanes, des atomes de fluors, des radicaux aryles, aryloxy, des acyles d'hydrocarbyles en C₁ à C₄₀ et des hydroxypropylénoxy.

Selon une variante particulière de l'invention, l'organopolysiloxane appartient à la famille des diméthicones-polyéthylèneglycols et notamment peut être choisi dans le groupe comprenant les diméthicones copolyol, en particulier la cétyldiméthicone copolyol et leurs dérivés. L'organopolysiloxane hydrophile selon la présente invention peut être le produit commercialisé sous la marque Abil WE09 ou Abil EM90 par la société Degussa-Goldschmidt. L'organopolysiloxane hydrophile selon la présente invention peut être également le produit commercialisé sous la référence KF-6017 par la société Shin-Etsu.

Le composé organopolysiloxane peut être en tout ou partie fluoré. En particulier, les groupements di-alkyles inférieurs siloxy peuvent être substitués par un ou plusieurs atomes de fluor.

Selon un mode particulier de réalisation, l'agent tensioactif siliconé utilisable dans les compositions cosmétiques conformes à la présente invention est choisi parmi la diméthicone copolyol, la diméthicone copolyol benzoate, les diméthicones copolyols phosphates, les élastomères de silicone polyoxyalkylénés, le mélange cyclométhicone/diméthicone et leurs mélanges.

Conviennent également selon l'invention les élastomères de silicones polyoxyalkylénés tels que ceux décrits dans les brevets US5236986, US5412004, US5837793, US5811487 dont le contenu est incorporé par référence.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société SHIN ETSU, "DC9010", "DC9011" par la société DOW CORNING.

Il est entendu que les compositions selon l'invention peuvent en outre comprendre des agents tensioactifs non siliconés anioniques et/ou non ioniques.

Pour le choix de ces agents tensioactifs, on peut se reporter au document "Encyclopedia of Chemical Technology, KIRK-OTHMER", volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs pouvant être plus particulièrement utilisés dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

### Matières colorantes

Selon un mode de réalisation, la composition selon l'invention peut contenir en outre au moins une matière colorante, organique ou inorganique, notamment de type pigments ou nacres.

Selon un autre mode de réalisation, la composition selon l'invention peut contenir en outre au moins une matière colorante choisie parmi les colorants lipophiles, les colorants hydrophiles, les pigments, les nacres, les matériaux à effet optique spécifique, et leurs mélanges.

Cette matière colorante peut être présente à raison de 0,01 à 50 % en poids par rapport au poids total de la composition, en particulier de 0,5 à 40 % et plus particulièrement de 5 à 25 %, et notamment de 0,01 à 20 %, et en particulier de 0,1 à 10 %, voire de 2 à 5 % en poids par rapport au poids total de la composition.

Par "pigments", il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 5 % en poids, et en particulier de 0,02 à 7 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF® NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL® PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA® , FLAMENCO® et DUOCHROME® (sur base de mica) commercialisées par la société ENGELHARD, les nacres TIMIRON® commercialisées par la société MERCK, les nacres sur base de mica PRESTIGE® commercialisées par la société ECKART et les nacres sur base de mica synthétique SUNSHINE® commercialisées par la société SUN CHEMICAL.

Les nacres peuvent plus particulièrement posséder une couleur ou un reflet jaune, rose, rouge, bronze, orangé, brun, or et/ou cuivré.

Les pigments peuvent être ou non enrobés superficiellement, en particulier traités en surface par des silicones, des acides aminés, des dérivés fluorés ou toute autre substance favorisant la dispersion et la compatibilité du pigment dans la composition.

Avantageusement, les pigments utilisés dans les compositions conformes à l'invention peuvent également être enrobés superficiellement par un revêtement de lécithine. Ce revêtement peut être obtenu par mise en contact d'une solution de pigment avec une solution de lécithine, en présence de sels de métaux di- ou trivalents. Pour obtenir ce revêtement, de la lécithine hydrogénée ou non hydrogénée peut être utilisée.

La composition cosmétique selon l'invention peut comporter également des colorants hydrosolubles ou liposolubles en une teneur allant de 0,01 à 10 % en poids, notamment allant de 0,01 à 5 % en poids par rapport au poids total de la composition cosmétique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Lorsque les compositions cosmétiques selon l'invention comportent un colorant hydrosoluble, ce dernier peut être présent dans la composition à l'état dispersé.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, "stabilisé" signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière
ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par "dérivés métalliques", on entend désigner des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE® 700 de la société ECKART, les particules d'aluminium enrobé de silice commercialisées sous la dénomination VISIONAIRE BRIGHT SILVER® de la société ECKART et les particules d'alliage métallique comme des poudres de bronze (alliage cuivre et zinc) enrobé de silice commercialisées sous la dénomination de VISIONAIRE BRIGHT NATURAL GOLD® de la société ECKART.

Il peut encore s'agir de particules comportant un substrat de verre comme celles commercialisées par la société NIPPON SHEET GLASS sous les dénominations MICROGLASS METASHINE® .

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA® par la société MERCK (Darmstadt). A titre d'exemple, ces pigments peuvent être les pigments de structure silice/oxyde de titane/oxyde d'étain commercialisés sous le nom XIRONA MAGIC® par la société MERCK, les pigments de structure silice/oxyde de fer brun commercialisés sous le nom XIRONA INDIAN SUMMER® par la société MERCK et les pigments de structure silice/oxyde de titane/mica/oxyde d'étain commercialisés sous le nom XIRONA CARRIBEAN BLUE® par la société MERCK. On peut encore citer les pigments INFINITE COLORS® de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

### AUTRES ADDITIFS

La composition de l'invention peut comprendre, en outre, tout ingrédient usuellement utilisé dans le domaine concerné.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

Ainsi, la composition selon l'invention comprend en outre des particules solides choisies parmi les charges.

La composition selon l'invention peut se présenter sous la forme d'une composition dermatologique ou de soin des matières kératiniques comme la peau ou les lèvres, sous forme d'une composition de protection solaire, de démaquillant sous forme de stick ou sous forme coulée. Elle peut notamment être utilisée comme base de soin pour la peau ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un blush, un fard à joues ou à paupières, un produit de maquillage du corps ; de maquillage des lèvres comme un rouge à lèvres, un brillant à lèvres ou un crayon, présentant éventuellement des propriétés de soin ou de traitement ; de maquillage des ongles, ou des cheveux notamment sous forme de crayon. Elle peut notamment se présenter sous la forme d'un stick anhydre.

Bien entendu, la composition de l'invention doit être cosmétiquement ou dermatologiquement acceptable, à savoir contenir un milieu physiologiquement acceptable non toxique et susceptible d'être appliqué sur la peau ou les lèvres d'êtres humains. Par cosmétiquement acceptable, on entend au sens de l'invention une composition d'aspect, d'odeur et de toucher agréables.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le(s) huile(s), puis à mélanger le tout. On ajoute alors, sous agitation, les matières colorantes, et/ou les particules solides et les additifs. Le mélange homogène obtenu peut alors être coulé dans un moule approprié comme un moule de rouge à lèvres ou directement dans les articles de conditionnement (boîtier ou coupelle notamment).

Selon un autre aspect, l'invention concerne également un ensemble cosmétique comprenant :
i) un récipient délimitant au moins un compartiment, ledit récipient étant fermé par un élément de fermeture ; et
ii) une composition disposée à l'intérieur dudit compartiment, la composition étant conforme à l'invention.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

L'invention va maintenant être décrite en référence aux exemples suivants, donnés à titre illustratif et non limitatif

### Exemple 1

### Formulation de rouge à lèvres

| INGREDIENTS | % en poids |
|---|---|
| Polymère Polyamide/polydiméthylsiloxane (n = 100) DC 2-8179 de Dow Corning | 25,00 |
| Parfum | 0,40 |
| Oxydes de fer brun jaune | 1,00 |
| Oxydes de fer noir | 0,47 |
| Dioxide de titane | 0,57 |
| Phényl triméthicone | 28,00 |
| Red 7 | 1,10 |
| Isononanoate d'isononyle | 20,00 |
| Polyisobutène hydrogéné | 17,46 |
| Cétyl PEG/PPG-10/1 diméthicone | 3,00 |
| Cire de polyéthylène | 3,00 |

La formule considérée testée selon les protocoles décrits précédemment présente les caractéristiques de texture suivantes :
- dureté de 74 g
- élasticité de 98 %.

### Exemple 2

### Formulation de rouge à lèvres.

| INGREDIENTS | | % en poids |
|---|---|---|
| Parfum | Parfum | 0,30 |
| Oxydes de fer brun, jaune (75/25) (CI : 77491 + 77492) | Colorant | 1,00 |
| Oxydes de fer noir (CI : 77499) | Colorant | 0,47 |
| Isoparaffine (6-8 moles d'isobutylène) hydrogénée | Corps gras | 18,10 |
| Phényl triméthylsiloxy trisiloxane (viscosité : 20 CST-PM : 372) | Silicone | 37,73 |
| Sel de calcium du rouge lithol B | Colorant | 1,10 |
| Polydiméthylsiloxane (viscosité: 5 CST) | Silicone | 11,53 |
| Poly méthylcétyl diméthyl méthylsiloxane oxyéthyléné (20/75/5 - viscosité : 3000 CST) | Silicone | 3,00 |
| Oxyde de titane rutile traité alumine/silice/triméthyolpropane (CI : 77891) | Colorant | 0,57 |
| Polyamide/Polydiméthylsiloxane de l'exemple 3 du brevet US 5 981 680 (n = 15) | Silicone | 1,20 |
| Polyamide/Polydiméthylsiloxane DC 2-8179 de Dow Corning (n = 100) | Silicone | 25,00 |

La formule considérée testée selon les protocoles décrits précédemment présente les caractéristiques de texture suivantes :
- dureté de 108 g
- élasticité de 97 %.

### Exemple 3

### Produit de maquillage

| Ingrédients | % en poids |
|---|---|
| Copolymère tribloc styrène/éthylène-butylène/styrène (Kraton G-1650) | 8,000 |
| Isoparaffine (6-8 moles d'isobutylène) hydrogénée (Polysinlane) | 77,860 |
| Oxyde de titane rutile traité alumine/silice/triméthyolpropane (CI : 77891) | 0,570 |
| Sel de calcium du rouge lithol B 1,100 | 1,100 |
| Oxyde de fer noir (CI : 77499) | 0,470 |
| Oxyde de fer brun, jaune (75/25) (CI : 77491 + 77492) | 1,000 |
| Isoparaffine (6-8 moles d'isobutylène) hydrogénée | 8,000 |
| Cire de polyéthylène (PM : 500) | 3,000 |
| | 100,000 |

La formule considérée testée selon les protocoles décrits précédemment présente les caractéristiques de texture suivantes :
- dureté de 30 g,
- élasticité de 99 %.

## Revendications

1. Composition cosmétique solide **caractérisée en ce qu'**elle comprend au moins une matière colorante et une phase grasse liquide texturée par une quantité efficace d'au moins un copolymère organique et **en ce qu'**elle présente une dureté variant de 10 à 250 g et une élasticité supérieure à 80 %.

2. Composition cosmétique solide **caractérisée en ce qu'**elle comprend une phase grasse liquide texturée par une quantité efficace d'au moins deux copolymères organiques distincts et **en ce qu'**elle présente une dureté variant de 10 à 250 g et une élasticité supérieure à 80 %.

3. Composition cosmétique solide **caractérisée en ce qu'**elle comprend une phase grasse liquide comprenant au moins une huile siliconée, ladite phase grasse liquide étant texturée par une quantité efficace d'au moins un copolymère organique et **en ce qu'**elle présente une dureté variant de 10 à 250 g et une élasticité supérieure à 80 %.

4. Composition cosmétique solide **caractérisée en ce qu'**elle comprend au moins un tensioactif siliconé et une phase grasse liquide texturée par une quantité efficace d'au moins un copolymère organique et **en ce qu'**elle présente une dureté variant de 10 à 250 g et une élasticité supérieure à 80 %.

5. Composition selon l'une quelconque des revendications précédentes, **caractérisée en ce que** ledit copolymère est choisi parmi :
1) des copolymères hybrides comportant au moins deux motifs capables d'établir des interactions hydrogène, ces deux motifs étant situés dans la chaîne du copolymère, notamment un polyorganosiloxane comportant au moins deux motifs capables d'établir des interactions hydrogènes, ces deux motifs étant situés dans la chaîne du copolymère, et/ou
2) des copolymères hybrides comportant au moins deux motifs capables d'établir des interactions hydrogènes, ces deux motifs étant situés sur des greffons ou ramifications, notamment un polyorganosiloxane comportant au moins deux motifs capables d'établir des interactions hydrogènes, ces deux motifs étant situés sur des greffons ou ramifications.

6. Composition selon la revendication 5, **caractérisée en ce que** lesdits motifs capables d'établir des interactions hydrogène sont choisis parmi les groupes ester, amide, sulfonamide, carbamate, thiocarbamate, urée, uréthane, thiourée, oxamido, guanidino, biguanidino et leurs combinaisons.

7. Composition selon l'une quelconque des revendications précédentes, dans laquelle ledit copolymère comprend au moins un motif répondant à la formule : 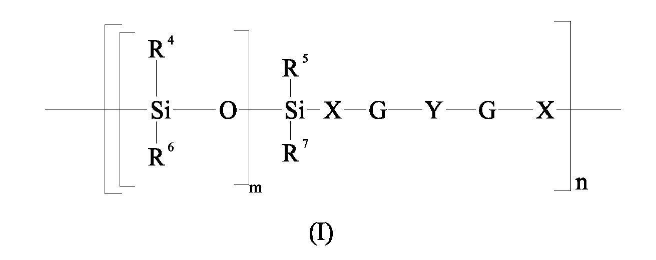 dans laquelle :
1) R⁴, R⁵, R⁶ et R⁷, identiques ou différents, représentent un groupe choisi parmi :
- les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C₆ à C₁₀, éventuellement substitués par un ou plusieurs groupes alkyle en C₁ à C₄,
- les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 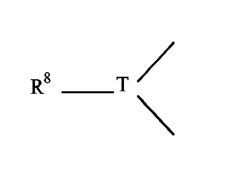 dans laquelle
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
- R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 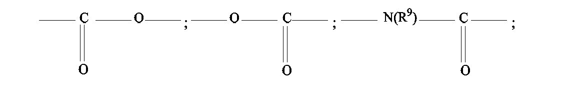 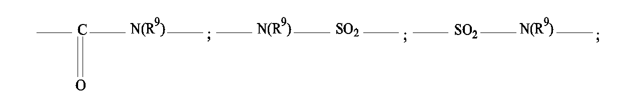 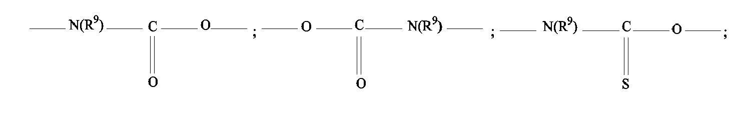 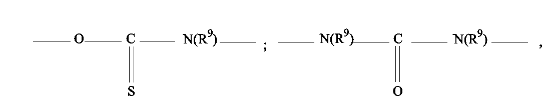 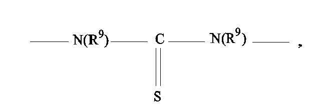 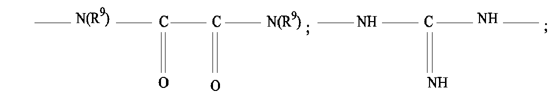 et 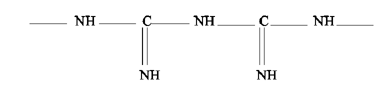 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 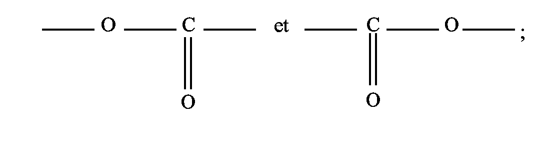
6) n est un nombre entier allant de 2 à 500, de préférence de 2 à 200, et m est un nombre entier allant de 1 à 1000, de préférence de 1 à 700 et mieux encore de 6 à 200.

8. Composition selon la revendication 7, dans laquelle Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆, et
g) les chaînes polyorganosiloxane de formule : 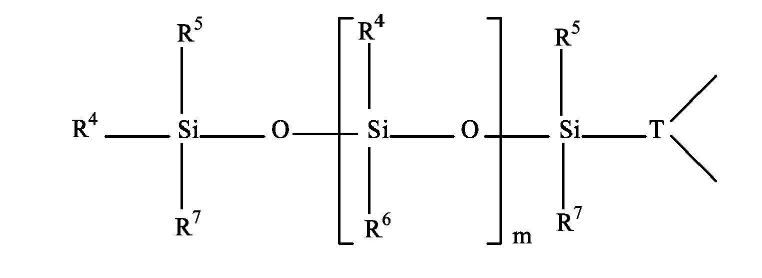
dans laquelle R⁴, R⁵, R⁶, R⁷, T et m sont tels que définis dans la revendication 7.

9. Composition selon l'une quelconque des revendications 1 à 8, dans laquelle le copolymère comprend au moins un motif de formule (III) ou (IV) : 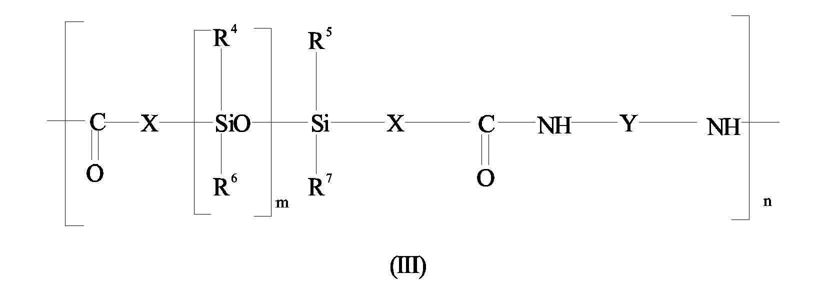 ou 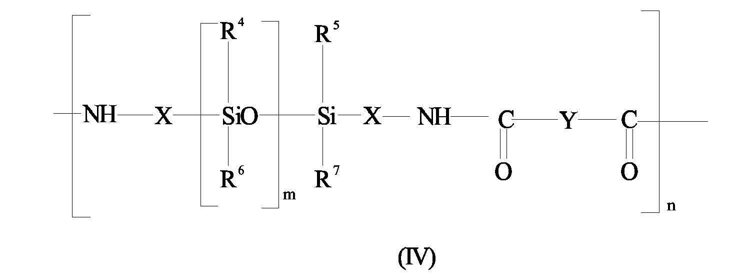 dans lesquelles R⁴, R⁵, R⁶, R⁷, X, Y, m et n sont tels que définis dans la revendication 7.

10. Composition selon la revendication 9, dans laquelle X et/ou Y représentent un groupe alkylène contenant dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃, et/ou substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

11. Composition selon l'une quelconque des revendications 9 ou 10, dans laquelle Y représente : 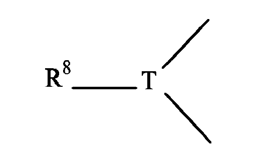 où R⁸ représente une chaîne polyorganosiloxane, et T représente un groupe de formule : 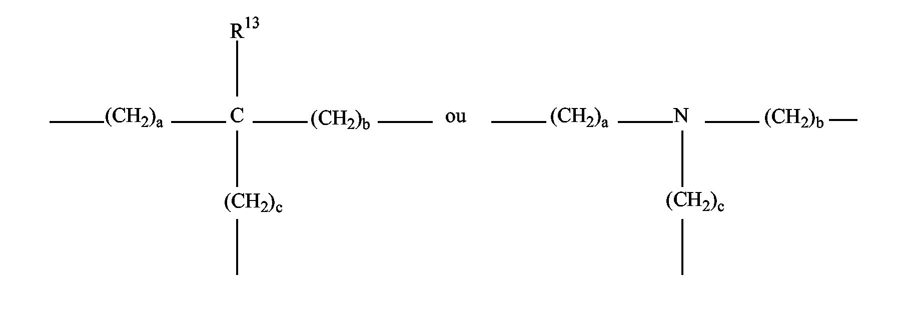 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷, dans la revendication 7.

12. Composition selon l'une quelconque des revendications 9 à 11, dans laquelle R⁴, R⁵, R⁶ et R⁷ représentent, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

13. Composition selon l'une quelconque des revendications 1 à 12, dans laquelle le copolymère comprend au moins un motif de formule (VII) : 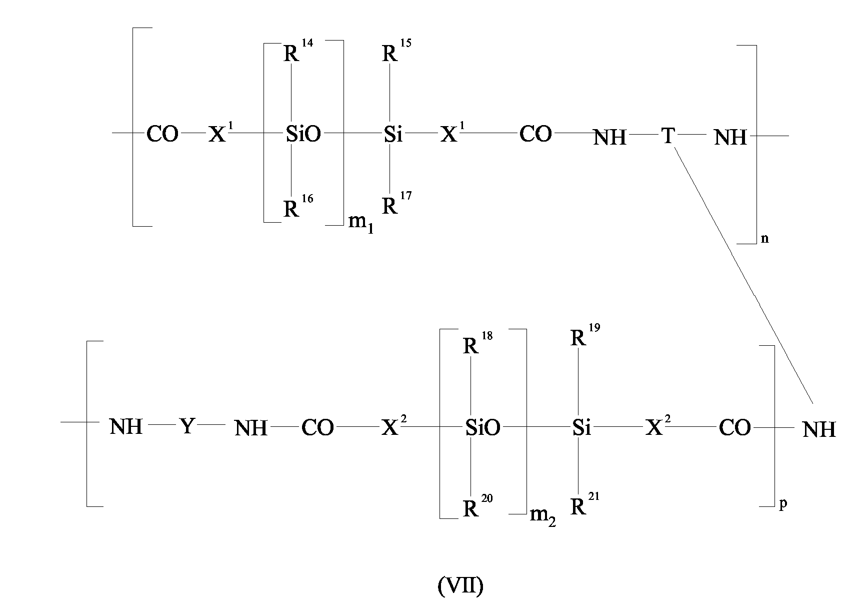 dans laquelle X¹ et X² qui sont identiques ou différents, ont la signification donnée pour X dans la revendication 7, n, Y et T sont tels que définis dans la revendication 7, R¹⁴ à R²¹ sont des groupes choisis dans le même groupe que les R⁴ à R⁷ de la revendication 7 m₁ et m₂ sont des nombres situés dans la gamme allant de 1 à 1 000, et p est un nombre entier allant de 2 à 500.

14. Composition selon la revendication 13 dans laquelle :
- p est dans la gamme de 1 à 25, mieux encore de 1 à 7,
- R¹⁴ à R²¹ sont des groupes méthyle,
- T répond à l'une des formules suivantes : 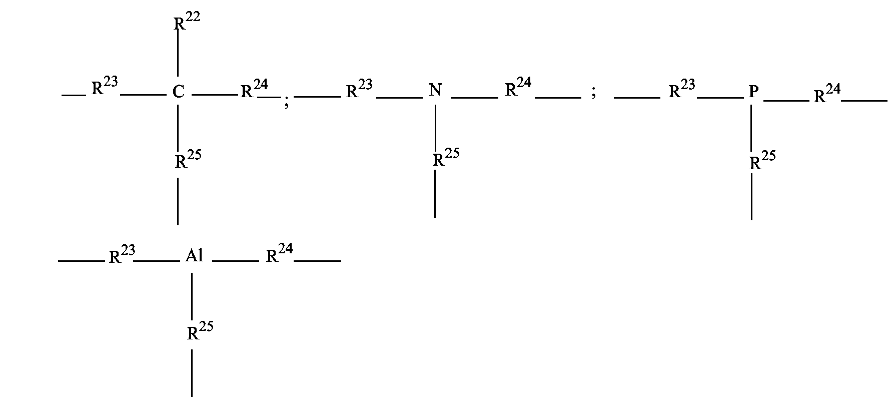
dans lesquelles R²² est un atome d'hydrogène ou un groupe choisi parmi les groupes définis pour R⁴ à R⁷, et R²³, R²⁴ et R²⁵ sont indépendamment des groupes alkylène, linéaires ou ramifiés, de préférence encore, à la formule : 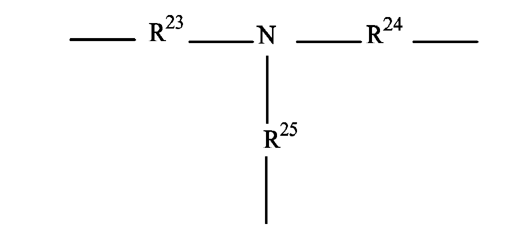 en particulier avec R²³, R²⁴ et R²⁵ représentant -CH₂-CH₂-,
- m₁ et m₂ sont dans la gamme de 15 à 500, et mieux encore de 15 à 45,
- X₁ et X₂ représentent -(CH₂)₁₀-, et
- Y représente -CH₂-.

15. Composition selon l'une quelconque des revendications 1 à 14 dans laquelle le copolymère comprend au moins un motif répondant à la formule suivante (VIII) : 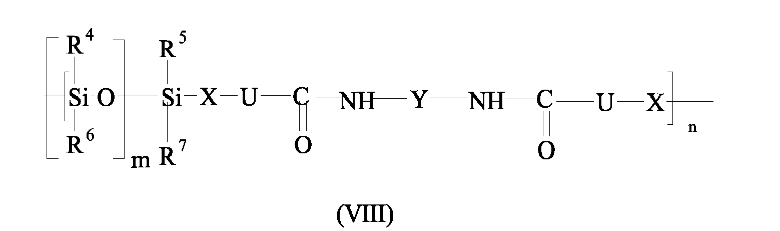 dans laquelle les R⁴, R⁵, R⁶, R⁷, X, Y, m et n ont les significations données ci-dessus pour la formule (I) dans la revendication 7 et U représente -O- ou -NH-, afin que : 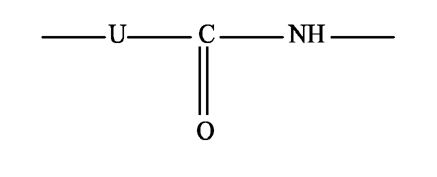 corresponde à un groupe uréthane ou urée, ou
Y représente un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane, ou Y représente un radical alkylène en C₁ à C₄₀, linéaire ou ramifié, ou un radical cycloalkylène en C₄ à C₁₂, ou
Y représente une séquence polyuréthane ou polyurée correspondant à la condensation de plusieurs molécules de diisocyanate avec une ou plusieurs molécules de coupleurs du type diol ou diamine, répondant à la formule (IX) : 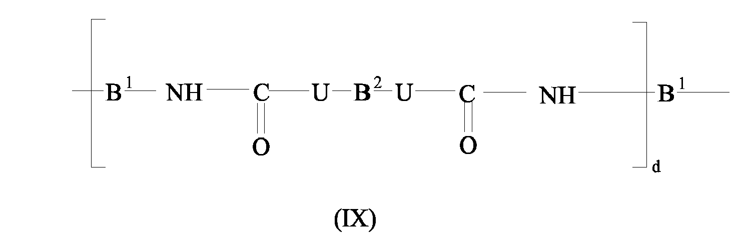 dans laquelle B¹ est un groupe choisi parmi les groupes donnés ci-dessus pour Y, U est -O- ou -NH-, et B² est choisi parmi :
• les groupes alkylène en C₁ à C₄₀, linéaires ou ramifiés,
• les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
• les groupes phénylène pouvant éventuellement être porteurs de substituants alkyles en C₁ à C₃, et
• les groupes de formule : 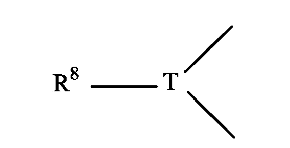
dans laquelle T est un radical trivalent hydrocarboné pouvant contenir un ou plusieurs hétéroatomes tels que l'oxygène, le soufre et l'azote et R⁸ est une chaîne polyorganosiloxane ou une chaîne alkyle en C₁ à C₅₀, linéaire ou ramifiée.

16. Composition cosmétique selon l'une quelconque des revendications précédentes, **caractérisée en ce que** le copolymère est choisi parmi les polymères comprenant au moins un motif répondant à la formule (II) : 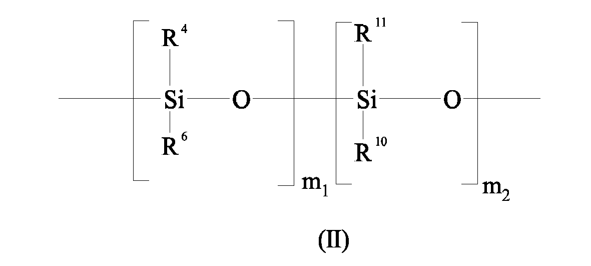 dans laquelle
R⁴ et R⁶, identiques ou différents, sont tels que définis ci-dessus pour la formule (I) en revendication 7,
R¹⁰ représente un groupe tel que défini ci-dessus pour R⁴ et R⁶, ou représente le groupe de formule -X-G-R¹² dans laquelle X et G sont tels que définis ci-dessus pour la formule (I) et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
R¹¹ représente le groupe de formule -X-G-R¹² ans laquelle X, G et R¹² sont tels que définis ci-dessus,
m₁ est un nombre entier allant de 1 à 998, et
m₂ est un nombre entier allant de 2 à 500.

17. Composition selon l'une quelconque des revendications précédentes, **caractérisée en ce que** le copolymère texturant est choisi parmi :
- les polyamides de formule (III) telle que définie en revendications 9 à 14 où m varie de 50 à 600, en particulier de 60 à 400, notamment de 75 à 200 et plus particulièrement est de l'ordre de 100 ;
- des mélanges de polyamide de formule (III) combinant :
1) 80 à 99 % en poids d'un polyamide où m varie de 50 à 600, en particulier de 60 à 400, notamment de 75 à 200 et plus particulièrement est de l'ordre de 100 et
2) 1 à 20 % d'un polyamide où m est dans la gamme de 5 à 100, en particulier de 10 à 75 et plus particulièrement est de l'ordre de 15;
- des mélanges de polyamide de formule (III) combinant :
1) 80 à 99 % en poids d'un polyamide où n varie de 2 à 10, en particulier de 3 à 6, et
2) 1 à 20 % d'un polyamide où n est dans la gamme de 5 à 500, en particulier de 30 à 100 ;
- des mélanges de polyamide de formule (III) combinant :
1) 1 à 20 % en poids d'un polyamide, où n est égal à 2 à 10, en particulier de 3 à 6, et
2) 80 à 99 % d'un polyamide, où n est dans la gamme de 30 à 500, en particulier de 30 à 100 ;
- des polyamides de formule (III) où X représente un radical alkyle en C₃ à C₁₅ en particulier en C₅ à C₁₂ et notamment en C₁₀ et
- des polyamides de formule (III) où Y représente un radical alkyle en C₃ à C₁₀ en particulier en C₄ à C₈ et notamment en C₆.

18. Composition selon l'une quelconque des revendications précédentes **caractérisée en ce qu'**elle comprend au moins un copolymère bloc polydiméthylsiloxane de formule générale I telle que définie selon l'une quelconque des revendications 7 à 15 et possédant un indice m de valeur supérieure à 50, en particulier supérieure à 75 et notamment d'environ 100.

19. Composition selon la revendication 18, **caractérisée en ce qu'**elle comprend en outre au moins un copolymère bloc polydiméthylsiloxane de formule générale I telle que définie selon l'une quelconque des revendications 7 à 15 possédant un indice m de valeur 15.

20. Composition cosmétique solide **caractérisée en ce qu'**elle comprend au moins une phase grasse liquide texturée par au moins deux copolymères de formule générale I telle que définie selon l'une quelconque des revendications 7 à 15 et 18 à 19, les deux copolymères possédant des indices m de valeurs différentes, l'une étant inférieure ou égale à 50 et l'autre supérieure ou égale à 50.

21. Composition cosmétique solide selon la revendication précédente **caractérisée en ce qu'**elle comprend :
- 1 à 90 % en poids d'au moins une huile siliconée,
- 5 à 50 % en poids en copolymère(s) possédant un indice m de valeur égale ou supérieure à 50, et notamment égale à 100,
- 0,1 à 10 % en poids en copolymère(s) possédant un indice m de valeur inférieure ou égale à 50, et notamment égale à 15,
- 0,1 à 10 % en poids d'un tensioactif siliconé, et
- 0,001 à 40 % en poids d'au moins une matière colorante.

22. Composition selon la revendication 20 ou 21, **caractérisée en ce qu'**elle présente une dureté variant de 10 à 250 g et une élasticité supérieure à 80 %.

23. Composition cosmétique solide de maquillage des lèvres **caractérisée en ce qu'**elle comprend au moins une phase grasse liquide texturée par une quantité efficace d'au moins un copolymère de formule générale I telle que définie selon l'une quelconque des revendications 7 à 15 et 18 à 19 et dans laquelle m est supérieur ou égal à 50.

24. Composition cosmétique solide **caractérisée en ce qu'**elle comprend au moins une phase grasse liquide texturée par une quantité efficace d'au moins un copolymère éthylénique et **en ce qu'**elle présente une dureté variant de 10 à 250 g et une élasticité supérieure à 80 %.

25. Composition selon la revendication 24, **caractérisée en ce que** le copolymère résulte de la copolymérisation d'un monomère éthylénique, en particulier en utilisant des copolymères vinyliques, acryliques ou méthacryliques.

26. Composition selon la revendication 24 ou 25, **caractérisée en ce que** ledit copolymère comprend un bloc styrène (S) ou un bloc alkylstyrène (AS), et un bloc choisi parmi les blocs éthylène/butylène (EB), éthylène/propylène (EP), butadiène (B), isoprène (I), acrylate (A), méthacrylate (MA) ou une association de ces blocs.

27. Composition selon la revendication 24, 25 ou 26, **caractérisée en ce que** ledit copolymère comprend au moins un bloc styrène.

28. Composition selon l'une quelconque des revendications 24 à 27, **caractérisée en ce que** ledit copolymère est choisi parmi les copolymères blocs de type polystyrène/polyisoprène, polystyrène/polybutadiène, polystyrène/copoly(éthylènepropylène), polystyrène/copoly(éthylène/butylène), copolymères de styrène-méthacrylate, polystyrène-copoly-éthylène/propylène) ou polystyrène-copoly(éthylène/butylène).

29. Composition selon l'une quelconque des revendications précédentes, **caractérisée en ce que** sa dureté varie de 30 g à 200 g et notamment de 50 g à 170 g et plus particulièrement de 75 g à 110 g.

30. Composition selon l'une quelconque des revendications précédentes, **caractérisée en ce que** son élasticité est supérieure à 90 % et notamment est proche de 100%.

31. Composition selon l'une quelconque des revendications précédentes, **caractérisée en ce qu'**elle comprend de 0,5 à 80 % du poids total de la composition, de préférence de 2 à 60 % et mieux de 5 à 40 % en poids de copolymère(s) par rapport du poids total de la composition.

32. Composition selon l'une quelconque des revendications précédentes, **caractérisée en ce que** le rapport massique copolymère organique/phase grasse liquide de la composition est de 0,01 à 10, notamment de 0,05 à 5, en particulier de 0,1 à 2.

33. Composition selon l'une quelconque des revendications précédentes, **caractérisée en ce qu'**elle comprend au moins une huile siliconée ayant un point éclair égal ou supérieur à 40 °C et avantageusement supérieur à la température de ramollissement du polymère texturant.

34. Composition selon la revendication 33 dans laquelle l'huile siliconée est choisie dans le groupe constitué par les composés suivants : l'octyltriméthicone, l'hexyltriméthicone, le décaméthyl cyclopentasiloxane D5, l'octaméthyl cyclotétratasiloxane D4, le dodécaméthyl cyclohexasiloxane D4, le dodécaméthyl cyclohexasiloxane D6, l'heptaméthyl octyltrisiloxane, le décaméthyl tétrasiloxane, le dodécaméthyl pentasiloxane, le polydiméthylsiloxane de 1,5 cSt, le polydiméthylsiloxane de 2 cSt, le polyméthylsiloxane de 3 cSt, le polydiméthylsiloxane de 5 cSt, et leurs mélanges.

35. Composition selon l'une quelconque des revendications 1 à 34, **caractérisée en ce qu'**elle comprend au moins une huile siliconée non volatile.

36. Composition selon l'une quelconque des revendications 1 à 35, **caractérisée en ce que** la phase grasse liquide contient au moins 5 % et en particulier de 10 à 90 % en poids d'huile(s) siliconée(s).

37. Composition selon l'une quelconque des revendications précédentes, **caractérisée en ce qu'**elle comprend en outre au moins une huile ester choisie parmi les esters des acides monocarboxyliques avec les monoalcools et polyalcools.

38. Composition selon la revendication 37, **caractérisée en ce que** ledit ester répond à la formule suivante :
R₁-CO-O-R₂
où
- R₁ représente un radical alkyle linéaire ou ramifié de 1 à 40 atomes de carbone, de préférence de 7 à 19 atomes de carbone, comprenant éventuellement une ou plusieurs doubles liaisons éthyléniques, et éventuellement substitué,
- R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

39. Composition selon la revendication 37 ou 38, **caractérisée en ce que** l'huile ester est choisie parmi les composés suivants : l'isononanoate d'isononyle, l'octanoate de cétostéaryle, le myristate d'isopropyle, le palmitate d'éthyl-2-hexyle, le stéarate d'octyl 2-dodécyle, l'érucate d'octyl 2-dodécyle, l'isostéarate d'isostéaryle et les heptanoates, octanoates, décanoates ou ricinoléates d'alcools ou de polyalcools.

40. Composition selon l'une quelconque des revendications précédentes, **caractérisée en ce que** la phase grasse liquide comprend de 0,5 à 100 % en poids, en particulier de 1 à 80 %, notamment de 2 à 50 % et plus particulièrement de 2 à 40 % de la ou des huiles esters.

41. Composition selon l'une quelconque des revendications précédentes, **caractérisée en ce qu'**elle se présente sous la forme d'un stick anhydre.

42. Composition selon l'une quelconque des revendications 1 à 22 et 24 à 41, **caractérisée en ce qu'**elle se présente sous forme de fond de teint, de rouge à lèvres, de blush, de produit de maquillage du corps, de fard à paupières ou à joues, de produit anticemes.

43. Procédé cosmétique de soin, de maquillage ou de traitement des matières kératiniques des être humains, comprenant l'application sur les matières kératiniques d'une composition cosmétique selon l'une quelconque des revendications précédentes.

44. Support synthétique sur lequel est présent, en tout ou partie de sa surface, au moins une couche d'une composition selon l'une quelconque des revendications 1 à 43.

45. Utilisation d'au moins un copolymère organique en association avec au moins une phase grasse liquide pour la préparation d'une composition cosmétique solide présentant une dureté variant de 10 à 250 g et une élasticité supérieure à 80 %.

46. Utilisation selon la revendication 45, **caractérisée en ce que** ledit copolymère est tel que défini en revendications 5 à 28.

47. Utilisation d'un copolymère de formule générale (I) telle que définie en revendications 7 à 15 et 18 à 20, dans laquelle m possède une valeur supérieure ou égale à 50 dans une composition de maquillage des lèvres.

48. Ensemble cosmétique comprenant :
a) un récipient délimitant au moins un compartiment, ledit récipient étant fermé par un élément de fermeture ; et
b) une composition disposée à l'intérieur dudit compartiment, la composition étant conforme à l'une quelconque des revendications 1 à 42.

49. Ensemble cosmétique selon la revendication 48, **caractérisé en ce que** le récipient est formé, au moins pour partie, en au moins un matériau thermoplastique.

50. Ensemble cosmétique selon la revendication 48, **caractérisé en ce que** le récipient est formé, au moins pour partie, en au moins un matériau non thermoplastique, notamment en verre ou en métal.

51. Ensemble selon l'une quelconque des revendications 48 à 50, **caractérisé en ce qu'**en position fermée du récipient, l'élément de fermeture est vissé sur le récipient.

52. Ensemble selon l'une quelconque des revendications 48 à 50, **caractérisé en ce qu'**en position fermée du récipient, l'élément de fermeture est couplé au récipient autrement que par vissage, notamment par encliquetage, collage, ou soudage.

53. Ensemble selon l'une quelconque des revendications 48 à 52, **caractérisé en ce que** la composition est sensiblement à la pression atmosphérique à l'intérieur du compartiment.

54. Ensemble selon l'une quelconque des revendications 48 à 52, **caractérisé en ce que** la composition est pressurisée à l'intérieur du récipient.
